# EUROPEAN PATENT SPECIFICATION

(11) **EP 2 914 546 B1**
(45) Date of publication and mention of the grant of the patent: **10.07.2019**
(21) Application number: 13851309.8
(22) Date of filing: 05.11.2013
(51) Int. Cl.: C01B 21/24, A01N 59/00, A61K 33/00, A61M 16/12, A61M 16/00, A61M 16/16, A61M 16/10

(54) **DUAL PLATFORM SYSTEM FOR THE DELIVERY OF NITRIC OXIDE**
SYSTEM MIT ZWEI PLATTFORMSYSTEMEN ZUR FREISETZUNG VON STICKOXID
SYSTÈME À DOUBLE PLATES-FORMES POUR L'ADMINISTRATION D'OXYDE NITRIQUE

(30) Priority: 05.11.2012 US 201261722595 P
(43) Date of publication of application: 09.09.2015
(73) Proprietor: VERO Biotech LLC, Cocoa, FL 32926 (US)
(72) Inventor: FINE, David H., Cocoa Beach, FL 32932 (US); BROMBERG, Edward, Orlando, FL 32836 (US); GAMERO, Lucas, Oviedo, FL 32765 (US); DENTON, Ryan, Titusville, FL 32780 (US); VASQUEZ, Gregory, Cocoa, FL 32922 (US); JOHNSON, Bryan, Orlando, FL 32833 (US)
(74) Representative: D Young & Co LLP
(86) International application number: PCT/US2013/068415
(87) International publication number: WO 2014/071350

(56) References cited:
- WO-A1-2010/014818
- US-A1- 2004 081 580
- US-A1- 2006 207 594
- US-A1- 2006 266 096
- US-A1- 2010 043 789
- US-A1- 2010 150 786
- US-A1- 2012 125 328
- US-A1- 2012 240 927

## Description

### TECHNICAL FIELD

The invention relates to a system including at least two independent platforms for generating nitric oxide.

### BACKGROUND

Nitric oxide (NO), also known as nitrosyl radical, is a free radical that is an important signalling molecule. For example, NO can cause smooth muscles in blood vessels to relax, thereby resulting in vasodilation and increased blood flow through the blood vessel. These effects can be limited to small biological regions since NO can be highly reactive with a lifetime of a few seconds and can be quickly metabolized in the body.

Some disorders or physiological conditions can be mediated by inhalation of nitric oxide. The use of low concentrations of inhaled nitric oxide can prevent, reverse, or limit the progression of disorders which can include, but are not limited to, acute pulmonary vasoconstriction, traumatic injury, aspiration or inhalation injury, fat embolism in the lung, acidosis, inflammation of the lung, adult respiratory distress syndrome, acute pulmonary edema, acute mountain sickness, post cardiac surgery acute pulmonary hypertension, persistent pulmonary hypertension of a newborn, perinatal aspiration syndrome, haline membrane disease, acute pulmonary thromboembolism, heparin-protamine reactions, sepsis, asthma and status asthmaticus or hypoxia. Nitric oxide can also be used to treat chronic pulmonary hypertension, bronchopulmonary dysplasia, chronic pulmonary thromboembolism and idiopathic or primary pulmonary hypertension or chronic hypoxia.

Generally, nitric oxide can be inhaled or otherwise delivered to the individual's lungs. Providing a therapeutic dose of NO could treat a patient suffering from a disorder or physiological condition that can be mediated by inhalation of NO or supplement or minimize the need for traditional treatments in such disorders or physiological conditions. Typically, the NO gas can be supplied in a bottled gaseous form diluted in nitrogen gas (N₂). Great care should be taken to prevent the presence of even trace amounts of oxygen (O₂) in the tank of NO gas because the NO, in the presence of O₂, can be oxidized to nitrogen dioxide (NO₂). Unlike NO, the part per million levels of NO₂ gas can be highly toxic if inhaled and can form nitric and nitrous acid in the lungs.

### SUMMARY

In one aspect, a system for the delivery of nitric oxide can include a first platform including a first source of nitric oxide, a second platform including a second source of a nitric oxide, and a delivery line coupled to the first platform and the second platform.

In some embodiments, the system can include a controller configured to operate the first platform and the second platform simultaneously. Operating simultaneously can mean that the first platform and the second platform can both operate during a period of time. A period of time can be at least 1 minute, at least 2 minutes, at least 5 minutes, at least 10 minutes, at least 30 minutes, at least 1 hour or at least 6 hours. Operating simultaneously may not mean that the first platform and the second platform must both always be operating. For example, the first platform can operate for 10 minutes (from minute 0 to minute 10). The second platform can operate for 10 minutes; however, the second platform may operate from minute 5 to minute 15. As a result, for the first 5 minutes, only the first platform may be operating. Then, from minute 5 to minute 10, both the first platform and the second platform may be operating. From minute 10 to minute 15, only the second platform may be operating. The first platform and the second platform may also operate simultaneously for more than one period of time. For example, the first platform and the second platform can both be operating for a first period of time. For a second period of time, the first platform may be shut down (e.g. for calibration, repair, cleaning, etc.) while the second platform continues to operate. Then the first platform can operate simultaneously again with the second platform for a third period of time.

In some embodiments, the system can include one or more receptacles. A receptacle can include a support and a reducing agent that can be capable of converting a nitric oxide-releasing compound to nitric oxide. In some embodiments, a nitric oxide-releasing compound can include dinitrogen tetroxide, nitrogen dioxide or a nitrite ion. In some embodiments, a nitric oxide-releasing compound can also include a compound having a N₂O₂- functional group.

In some embodiments, a support can be any material that has at least one solid or non-fluid surface (e.g. a gel). A support can be porous or permeable. A support can be surface-active material, for example, a material with a large surface area that can be capable of retaining water or absorbing moisture. In some embodiments, a surface active material can include silica gel or cotton. The term "surface-active material" denotes that the material supports an active agent on its surface.

In some embodiments, a support can be covered or coated with a reducing agent. In some embodiments, a support can be saturated with an aqueous solution of a reducing agent. A reducing agent can include one or more compounds capable of donating an electron to another species during a reduction-oxidation (redox) reaction. A reducing agent can include hydroquinone, glutathione, and/or one or more reduced metal salts such as Fe(II), Mo(VI), NaI, Ti(III) or Cr(III), thiols, or NO₂⁻. A reducing agent can be safe (i.e., non-toxic and/or non-caustic) for inhalation by a mammal, for example, a human. A reducing agent can include an antioxidant. An antioxidant can be an aqueous solution of an antioxidant. An antioxidant can be ascorbic acid, alpha tocopherol, or gamma tocopherol. An antioxidant can be used dry or wet.

In some embodiments, a first platform can include at least one receptacle. In some embodiments, a first platform can include a first receptacle. A first receptacle can be coupled to a first source of nitric oxide. A first receptacle can include a first support and a first reducing agent capable of converting a first nitric oxide-releasing compound to nitric oxide.

In some embodiments, a second platform can include at least one receptacle. In some embodiments, a second platform can include a second receptacle. A second receptacle can be coupled to a second source of nitric oxide. A second receptacle can include a second support and a second reducing agent capable of converting a second nitric oxide-releasing compound to nitric oxide.

In some embodiments, a first reducing agent and a second reducing agent can include the same reducing agent(s).

In some embodiments, a delivery line can include at least one receptacle.

In some embodiments, a first source of nitric oxide can include a first nitric oxide-releasing compound. In some cases, a first nitric oxide-releasing compound can include nitrogen dioxide and/or nitric oxide. In some cases, a second nitric oxide-releasing compound can include nitrogen dioxide and/or nitric oxide. In some embodiments, a first nitric oxide-releasing compound and a second nitric oxide-releasing compound can include the same compound.

In some embodiments, a first source of nitric oxide and/or a second source of nitric oxide can include a gas bottle. A gas bottle can be pressurized. Pressurized can mean that the gas in the bottle is kept at a pressure greater than atmospheric pressure. In some circumstances, a gas bottle can be called a tank or a gas tank.

In some embodiments, a first source of nitric oxide and/or a second source of nitric oxide can include a reservoir. A reservoir can include a nitric oxide-releasing oxide. In some embodiments, a reservoir can also include nitrogen dioxide vapor or nitrogen dioxide gas in a space over the nitrogen dioxide source.

In some embodiments, a nitric oxide-releasing oxide can be dinitrogen tetroxide, more specifically, liquid dinitrogen tetroxide. The amount of nitric oxide-releasing oxide in a reservoir can be less than about 5.0 g, less than about 2.0 g, less than about 1.0 g, less than about 0.50 g, less than 0.25 g or less than 0.10 g; the amount of nitric oxide-releasing oxide in a reservoir can be greater than about 0.05 g, greater than about 0.10 g, greater than about 0.20 g, greater than about 0.50 g or greater than about 1.0 g. The amount of nitric oxide-releasing oxide in a reservoir can be less than about 5 ml, less than about 2 ml, less than about 1 ml, less than about 0.5 ml, less than about 0.25 ml or less than about 0.10 ml; the amount of nitric oxide-releasing oxide in a reservoir can be greater than about 0.001 ml, greater than about 0.01 ml, greater than about 0.05, greater than about 0.10 ml, greater than about 0.25ml, greater than about 0.50 ml or greater than about 1.0 ml.

In some embodiments, a reservoir can include one or more restrictors. In some cases, each reservoir can include two or more restrictors. In some embodiments, a restrictor can be coupled to a reservoir.

In some embodiments, a restrictor can be an orifice. In some embodiments, the restrictor can be a tube. In some embodiments, the tube can be a capillary tube, more specifically, a quartz capillary tube.

In some embodiments, a restrictor can include a first end and a second end. In some embodiments, a first end of a restrictor can be coupled to a reservoir. In some embodiments, a second end can be sealed or closed. In some embodiments, a second end, which was previously sealed or closed, can be opened, unsealed or include a broken seal. In some embodiments, a second end of a restrictor can also be coupled to a first platform or a second platform. In some embodiments, a first platform or a second platform can include a device for opening a second end or breaking a seal on a second end.

A restrictor can have a length. In some embodiments, a restrictor can further include a length corresponding to the distance between the first end and the second end. In some embodiments, the length of a first restrictor can substantially identical to the length of a second restrictor. In other embodiments, the length of a first restrictor can be different than the length of a second restrictor.

In some embodiments, the length of a restrictor can be at least about 0.1 inch, at least about 0.25 inch or at least about 0.5 inch; the length can be at most about 4 inches, at most about 2 inches, at most about 1 inch, or at most about 0.5 inch. Preferably, a restrictor can have a length of about 0.75 inch.

A restrictor can have an internal diameter. In some embodiments, the internal diameter of a first restrictor can be substantially identical to the internal diameter of a second restrictor. In other embodiments, the internal diameter of a first restrictor can be different than the internal diameter of a second restrictor. In some embodiments, an internal diameter of a restrictor can be at least about 1, at least about 5 microns or at least about 10 microns; an internal diameter can be at most about 100 microns, at most about 50 microns, at most about 25 microns, or at most about 10 microns. Preferably, the restrictor can have a diameter of about 10 microns.

In some embodiments, a first restrictor can be configured to release a first amount of nitric oxide-releasing compound. In some embodiments, a second restrictor can be configured to release a second amount of nitric oxide-releasing compound. In some cases, a first amount of nitric oxide-releasing compound can be greater than a second amount of nitric oxide-releasing compound. More specifically, in some cases, a first amount of nitric oxide-releasing compound can be at least 2 times, at least 4 times or at least 9 times greater than a second amount of nitric oxide-releasing compound.

In some embodiments, a restrictor can include a valve. In some cases, a valve can have an open position that can allow a gas to pass through the restrictor. In some cases, a valve can have a closed position that can prevent a gas from passing through the restrictor.

In some embodiments, a controller can be configured to independently switch a valve on a restrictor between the open position and the closed position. In some embodiments, a controller can be configured to independently switch a valve on each restrictor between the open position and the closed position.

In some embodiments, a controller can be configured to receive a temperature input representing a first predetermined temperature at which a first reservoir should operate. In some embodiments, a first platform can include a first heating device. A first heating device can be thermally connected with a reservoir of a first source of nitric oxide and/or one or more restrictors of the reservoir of the first source of nitric oxide.

In some embodiments, a controller can be configured to receive a temperature input representing a second predetermined temperature at which a second reservoir should operate. In some embodiments, a second platform can include a second heating device. A second heating device can be thermally connected with a reservoir of a second source of nitric oxide and/or one or more restrictors of the reservoir of the second source of nitric oxide.

In some embodiments, a system can include a first gas in the first platform. The first gas can include nitric oxide. The first platform can be configured to deliver the first gas to the delivery line. In some embodiments, a system can include a second gas in the second platform. The second gas can include nitric oxide. The second platform can be configured to deliver the second gas to the delivery line.

In some embodiments, the amount of nitric oxide in the first gas may not be equal to the amount of nitric oxide in the second gas. In some cases, the amount of nitric oxide in the first gas can be at least twice, at least four times, or at least nine times the amount of nitric oxide in the second gas.

In some embodiments, a sensor module can include one or more nitric oxide sensors. In some embodiments, a controller can be configured to receive a nitric oxide detection signal from at least one of the one or more nitric oxide sensors. In some embodiments, a sensor module can include a first sensor line including one or more nitric oxide sensors. In some embodiments, a controller can be configured to receive a first nitric oxide detection signal from at least one of the one or more nitric oxide sensors in a first sensor line. In some embodiments, a second sensor line can include one or more nitric oxide sensors. In some embodiments, a second nitric oxide detection signal from at least one of the one or more nitric oxide sensors in a second sensor line.

In some embodiments, a sensor module can be coupled to a delivery line. In some embodiments, a controller can be configured to receive a nitric oxide-signal representing a predetermined amount of nitric oxide to be delivered via a delivery line. A controller can be configured to compare each of a first nitric oxide detection signal and a second nitric oxide detection signal to a nitric oxide-signal. In some cases, a controller can be configured to activate an alert if a first nitric oxide detection signal or a second nitric oxide detection signal differ from the nitric oxide-signal by greater than 2%, greater than 5%, greater than 10%, greater than 15% or greater than 20% of the nitric oxide-signal. A controller can be configured to activate an alert if a first nitric oxide detection signal and a second nitric oxide detection signal differ by greater than 2%, greater than 5%, greater than 10%, greater than 15% or greater than 20% of the first nitric oxide detection signal.

In some embodiments, a sensor module can include one or more nitrogen dioxide sensors. In some embodiments, a controller can be configured to receive a nitrogen dioxide detection signal from at least one of the one or more nitrogen dioxide sensors. In some embodiments, a sensor module can include a first sensor line including one or more nitrogen dioxide sensors. In some embodiments, a controller can be configured to receive a first nitrogen dioxide detection signal from at least one of the one or more nitrogen dioxide sensors in a first sensor line. In some embodiments, a second sensor line can include one or more nitrogen dioxide sensors. In some embodiments, a second nitrogen dioxide detection signal from at least one of the one or more nitrogen dioxide sensors in a second sensor line.

In some embodiments, a controller can be configured to receive a nitrogen dioxide-signal representing a predetermined amount of nitrogen dioxide that can be present in a delivery line. A controller can be configured to compare each of a first nitrogen dioxide detection signal and a second nitrogen dioxide detection signal to a nitrogen dioxide-signal. In some cases, a controller can be configured to activate an alert if a first nitrogen dioxide detection signal or a second nitrogen dioxide detection signal differ from the nitrogen dioxide-signal by greater than 2%, greater than 5%, greater than 10%, greater than 15% or greater than 20% of the nitrogen dioxide-signal. A controller can be configured to activate an alert if a first nitrogen dioxide detection signal and a second nitrogen dioxide detection signal differ by greater than 2%, greater than 5%, greater than 10%, greater than 15% or greater than 20% of the first nitrogen dioxide detection signal.

In some embodiments, a sensor module can include one or more oxygen sensors. In some embodiments, a controller can be configured to receive an oxygen detection signal from at least one of the one or more oxygen sensors. In some embodiments, a sensor module can include a first sensor line including one or more oxygen sensors. In some embodiments, a controller can be configured to receive a first oxygen detection signal from at least one of the one or more oxygen sensors in a first sensor line. In some embodiments, a second sensor line can include one or more oxygen sensors. In some embodiments, a second oxygen detection signal from at least one of the one or more oxygen sensors in a second sensor line.

In some embodiments, a controller can be configured to receive an oxygen-signal representing a predetermined amount of oxygen that can be present in a delivery line. A controller can be configured to compare each of a first oxygen detection signal and a second oxygen detection signal to an oxygen-signal. In some cases, a controller can be configured to activate an alert if a first oxygen detection signal or a second oxygen detection signal differ from the oxygen-signal by greater than 2%, greater than 5%, greater than 10%, greater than 15% or greater than 20% of the oxygen-signal. A controller can be configured to activate an alert if a first oxygen detection signal and a second oxygen detection signal differ by greater than 2%, greater than 5%, greater than 10%, greater than 15% or greater than 20% of the first oxygen detection signal.

In some embodiments, a first platform can include a first sensor module including one or more nitric oxide sensors. In some embodiments, a second platform can include a second sensor module including one or more nitric oxide sensors. In some cases, a controller can be configured to receive a first nitric oxide detection signal from a nitric oxide sensor of the one or more nitric oxide sensors of the first platform, a second nitric oxide detection signal from a nitric oxide sensor of the one or more nitric oxide sensors of the second platform, and/or a nitric oxide-signal representing the predetermined amount of nitric oxide in the delivery line.

In some embodiments, a first sensor module can include one or more nitrogen dioxide sensors. In some embodiments, a second sensor module can include one or more nitrogen dioxide sensors. In some cases, a controller can be configured to receive a first nitrogen dioxide detection signal from a nitrogen dioxide sensor of the one or more nitrogen dioxide sensors of the first platform, a second nitrogen dioxide detection signal from a nitrogen dioxide sensor of the one or more nitrogen dioxide sensors of the second platform, and/or a nitrogen dioxide-signal representing the predetermined amount of nitrogen dioxide in the delivery line.

In some embodiments, a first sensor module can include one or more oxygen sensors. In some embodiments, a second sensor module can include one or more oxygen sensors. In some cases, a controller can be configured to receive a first oxygen detection signal from an oxygen sensor of the one or more oxygen sensors of the first platform, a second oxygen detection signal from an oxygen sensor of the one or more oxygen sensors of the second platform, and/or an oxygen-signal representing the predetermined amount of oxygen in the delivery line.

In some embodiments, a first platform can include a first flow valve prior to a delivery line. A first flow valve can have a first position configured to allow a first gas to pass from a first platform into a delivery line. A first flow valve can have a second position configured to allow a first gas to pass out of a first platform via a first dump line. In some embodiments, second platform can include a second flow valve prior to a delivery line. A second flow valve can have a first position configured to allow a second gas to pass from a second platform into a delivery line. A second flow valve can have a second position configured to allow a second gas to pass out of a second platform via a second dump line.

In some embodiments, the system can include a first flow valve in a first platform prior to a delivery line. A first flow valve can have an open position configured to allow a first gas to pass from a first platform into a delivery line. A first flow valve can have a closed position configured to prevent a first gas from passing from a first platform into a delivery line. In some embodiments, the system can include a second flow valve in a second platform prior to a delivery line. A second flow valve can have an open position configured to allow a second gas to pass from a second platform into a delivery line. A second flow valve can have a closed position configured to prevent a second gas from passing from a second platform into a delivery line.

In some embodiments, the system can include a first dump line coupled to a first platform by a first dump valve. A first dump valve can have an open position configured to allow a first gas to pass from a first platform into a first dump line. A first dump valve can have a closed position configured to prevent a first gas from passing into a first dump line. In some embodiments, the system can include a second dump line coupled to a second platform by a second dump valve. A second dump valve can have an open position configured to allow a second gas to pass from a second platform into a second dump line. A second dump valve can have a closed position configured to prevent the second gas from passing into the second dump line.

In some embodiments, the system can include a set of nitric oxide calibration standards including at least one nitric oxide calibration fluid. Each nitric oxide calibration fluid can have a different known amount of nitric oxide.

In some embodiments, a controller can be configured to switch a first flow valve from an open position to a closed position. In some cases, a controller can be configured to switch a first flow valve from an open position to a closed position if at least one of the one or more nitric oxide sensors in the first platform detects an amount of nitric oxide gas in one of the at least two nitric oxide calibration fluids that differs by 20% or more, 15% or more, 10% or more, 5% or more or 2% or more from the known amount of nitric oxide in the at least one nitric oxide calibration fluid. In some cases, a controller can be configured to switch an second valve from an open position to a closed position if at least one of the one or more nitrogen dioxide sensors in the second platform detects an amount of nitric oxide gas in one of the at least two nitric oxide calibration fluids that differs by 20% or more, 15% or more, 10% or more, 5% or more or 2% or more from the known amount of nitric oxide in the at least one nitric oxide calibration fluid.

In some embodiments, the system can include a set of nitrogen dioxide calibration standards including at least one nitrogen dioxide calibration fluids. Each nitrogen dioxide calibration fluid can have a different known amount of nitrogen dioxide.

In some embodiments, the system can include a set of oxygen calibration standards including at least one oxygen calibration fluids, each oxygen calibration fluid having a different known amount of oxygen.

In some embodiments, a delivery line can include a patient interface. A patient interface can include a mouth piece, nasal cannula, face mask, fully-sealed face mask or an endotracheal tube.

In some embodiments, a delivery line can include a ventilator.

In some embodiments, a delivery line can include a mixing receptacle. A mixing receptacle can include a support and a reducing agent capable of converting a nitric oxide-releasing compound to nitric oxide.

In another aspect, a method of delivering nitric oxide can include communicating a first gas including nitric oxide through a first platform to a delivery line. In some embodiments, the method of delivering nitric oxide can include communicating a second gas including nitric oxide through a second platform to the delivery line. In some embodiments, the method of delivering nitric oxide can include delivering the first gas and the second gas to a mammal via a delivery line. In some embodiments, a first gas can be communicated through a first platform at the same time (i.e. simultaneously, as discussed herein) as the second gas is communicated through the second platform.

In some embodiments, communicating a first gas including nitric oxide through a first platform can include releasing the first gas including a first nitric oxide-releasing compound from a first nitric oxide source into the first platform. A first platform can include a first receptacle including a first support and a first reducing agent. In some cases, communicating a first gas including nitric oxide through a first platform can include contacting the first nitric oxide-releasing compound in the first gas with the first reducing agent to generate nitric oxide.

In some embodiments, communicating a second gas including nitric oxide through a second platform can include releasing the second gas including a second nitric oxide-releasing compound from a second nitric oxide source into the second platform. A second platform can include a second receptacle including a second support and a second reducing agent. In some cases, communicating a second gas including nitric oxide through a second platform can include contacting the second nitric oxide-releasing compound in the second gas with the second reducing agent to generate nitric oxide.

In some embodiments, the amount of the first nitric oxide-releasing compound in the first gas may not be equal to the amount of the second nitric oxide-releasing compound in the second gas. In some cases, the amount of the first nitric oxide-releasing compound in the first gas can be at least twice, at least four times, or at least nine times the amount of the second nitric oxide-releasing compound in the second gas.

In some embodiments, a first nitric oxide-releasing compound can include dinitrogen tetroxide or nitrogen dioxide. In some embodiments, a second nitric oxide-releasing compound can include dinitrogen tetroxide or nitrogen dioxide. In some cases, the first nitric oxide-releasing compound and the second nitric oxide-releasing compound can include the same compound.

In some embodiments, a first source and/or second source of nitric oxide can include a gas bottle.

In some embodiments, a first source of nitric oxide can include a first reservoir. In some embodiments, a first reservoir can include one or more restrictors.

In some embodiments, releasing the first gas including the first nitric oxide-releasing compound from the first reservoir can include releasing a first amount of the first nitric oxide-releasing compound from the first reservoir via a first restrictor and a second amount of the first nitric oxide-releasing compound from the first reservoir via a second restrictor. In some cases, an amount can be a concentration, a mass or a volume. In some cases, the first amount of the first nitric oxide-releasing compound and the second amount of the first nitric oxide-releasing compound can be different amounts.

In some embodiments, releasing the first amount of the first nitric oxide-releasing compound from the first reservoir via the first restrictor can include opening a valve coupled to the first restrictor. In some embodiments, releasing the second amount of the first nitric oxide-releasing compound from the first reservoir via the second restrictor can include opening a valve coupled to the second restrictor.

In some embodiments, the second source of nitric oxide can include a second reservoir. In some cases, a second reservoir can include one or more restrictors.

In some embodiments, releasing the second gas including the second nitric oxide-releasing compound from the second reservoir can include releasing a first amount of the second nitric oxide-releasing compound from the second reservoir via a first restrictor and a second amount of the second nitric oxide-releasing compound from the second reservoir via a second restrictor. In some embodiments, the first amount of the second nitric oxide-releasing compound and the second amount of the second nitric oxide-releasing compound can be different amounts.

In some embodiments, releasing the first amount of the second nitric oxide-releasing compound from the second reservoir via the first restrictor can include opening a valve coupled to the first restrictor. In some embodiments, releasing the second amount of the second nitric oxide-releasing compound from the second reservoir via the second restrictor can include opening a valve coupled to the second restrictor.

In some embodiments, the method can include heating the first reservoir. In some cases, the method can include heating the first reservoir to a first predetermined temperature. In some embodiments, the method can include maintaining the first reservoir at the first predetermined temperature. In some embodiments, the first reservoir can be heated using a heating device that can be thermally connected with the first reservoir and the one or more restrictors of the first reservoir. In some embodiments, heating the first reservoir can increase a total amount of the first nitric oxide-releasing compound released from the first reservoir.

In some embodiments, the method can include heating the second reservoir. In some cases, the method can include heating the second reservoir to a second predetermined temperature. In some embodiments, the method can include maintaining the second reservoir at the second predetermined temperature. In some embodiments, the second reservoir can be heated using a heating device that can be thermally connected with the second reservoir and the one or more restrictors of the second reservoir. In some embodiments, heating the second reservoir can increase a total amount of the second nitric oxide-releasing compound released from the second reservoir.

In some embodiments, the method can include supplying a diluent gas into the delivery line. In some embodiments, delivering the first gas and the second gas to the mammal via the delivery line can include mixing the first gas, second gas and diluent gas together to form a delivery gas.

In some embodiments, the first gas, second gas and diluent gas are mixed together in a third receptacle in the delivery line. A third receptacle can include a third support and a third reducing agent.

In some embodiments, delivering the first gas and the second gas to the mammal via the delivery line can include communicating the delivery gas through the delivery line. In some embodiments, 50% or more, 75% or more, 80% or more, 90% or more, or 95% or more of the delivery gas can be diluent gas.

In some embodiments, the method can include detecting the amount of nitric oxide in the delivery gas using a nitric oxide sensor module. In some embodiments, a nitric oxide sensor module can include one or more nitric oxide sensors.

In some embodiments, the method can include comparing the amount of nitric oxide detected in the delivery gas with a predetermined amount of nitric oxide to be delivered to the mammal.

In some embodiments, the method can include activating an alert if the amount of nitric oxide detected in the delivery gas differs from the predetermined amount of nitric oxide by greater than 2%, greater than 5%, greater than 10%, greater than 15% or greater than 20% of the predetermined amount of nitric oxide.

In some embodiments, the method can include discharging at least a portion of the first gas from the first platform if the amount of nitric oxide detected in the delivery gas differs from the predetermined amount of nitric oxide by greater than 2%, greater than 5%, greater than 10%, greater than 15% or greater than 20% of the predetermined amount of nitric oxide.

In some embodiments, the method can include discharging at least a portion of the second gas from the second platform if the amount of nitric oxide detected in the delivery gas differs from the predetermined amount of nitric oxide by greater than 2%, greater than 5%, greater than 10%, greater than 15% or greater than 20% of the predetermined amount of nitric oxide.

In some embodiments, the method can include uncoupling the first platform from the delivery line if the amount of nitric oxide detected in the delivery gas differs from the predetermined amount of nitric oxide by greater than 2%, greater than 5%, greater than 10%, greater than 15% or greater than 20% of the predetermined amount of nitric oxide.

In some embodiments, the method can include uncoupling the second platform from the delivery line if the amount of nitric oxide detected in the delivery gas differs from the predetermined amount of nitric oxide by greater than 2%, greater than 5%, greater than 10%, greater than 15% or greater than 20% of the predetermined amount of nitric oxide.

In some embodiments, detecting the amount of nitric oxide in the delivery gas using the nitric oxide sensor module can include communicating a first portion of a sample of the delivery gas into a first sensor line including one or more nitric oxide sensors and a second portion of the sample into a second sensor line including one or more nitric oxide sensors.

In some embodiments, detecting the amount of nitric oxide in the delivery gas using the nitric oxide sensor module can include detecting an amount of nitric oxide in the first portion of the sample using at least one of the one or more nitric oxide sensors in the first sensor line and detecting an amount of nitric oxide in the second portion of the sample using at least one of the one or more nitric oxide sensors in the second sensor line.

In some embodiments, the method can include comparing the amount of nitric oxide detected in the first portion of the sample and/or the second portion of the sample with a predetermined amount of nitric oxide to be delivered to the mammal.

In some embodiments, the method can include activating an alert if the amount of nitric oxide detected in the first portion of the sample and/or the second portion of the sample differs from the predetermined amount of nitric oxide by greater than 2%, greater than 5%, greater than 10%, greater than 15% or greater than 20% of the predetermined amount of nitric oxide.

In some embodiments, the method can include discharging at least a portion of the first gas from the first platform if the amount of nitric oxide detected in the first portion of the sample and/or the second portion of the sample differs from the predetermined amount of nitric oxide by greater than 2%, greater than 5%, greater than 10%, greater than 15% or greater than 20% of the predetermined amount of nitric oxide.

In some embodiments, the method can include discharging at least a portion of the second gas from the second platform if the amount of nitric oxide detected in the first portion of the sample and/or the second portion of the sample differs from the predetermined amount of nitric oxide by greater than 2%, greater than 5%, greater than 10%, greater than 15% or greater than 20% of the predetermined amount of nitric oxide.

In some embodiments, the method can include uncoupling the first platform from the delivery line if the amount of nitric oxide detected in the first portion of the sample and/or the second portion of the sample differs from the predetermined amount of nitric oxide by greater than 2%, greater than 5%, greater than 10%, greater than 15% or greater than 20% of the predetermined amount of nitric oxide.

In some embodiments, the method can include uncoupling the second platform from the delivery line if the amount of nitric oxide detected in the first portion of the sample and/or the second portion of the sample differs from the predetermined amount of nitric oxide by greater than 2%, greater than 5%, greater than 10%, greater than 15% or greater than 20% of the predetermined amount of nitric oxide.

In some embodiments, detecting the amount of nitric oxide in the delivery gas using the nitric oxide sensor module can include comparing the amount of nitric oxide detected in the first portion of the sample with the amount of nitric oxide detected in the second portion of the sample. In some embodiments, detecting the amount of nitric oxide in the delivery gas using the nitric oxide sensor module can include activating an alert if the amount of nitric oxide detected in the first portion of the sample differs from the amount of nitric oxide detected in the second portion of the sample by more than 2%, more than 5%, more than 10%, more than 15% or more than 20% of the amount of nitric oxide detected in the first portion of the sample.

In some embodiments, a method can include detecting the amount of nitrogen dioxide in the delivery gas using a nitrogen dioxide sensor module including one or more nitrogen dioxide sensors. In some embodiments, a method can include detecting the amount of oxygen in the delivery gas using an oxygen sensor module including one or more oxygen sensors.

In some embodiments, a single sensor module can include the nitric oxide sensor module, the nitrogen dioxide sensor module and the oxygen sensor module.

In some embodiments, a method can include calibrating the one or more nitric oxide sensors.

In some embodiments, a method can include communicating a first portion of a sample of the delivery gas into a first sensor line including one or more nitric oxide sensors, detecting an amount of nitric oxide in the first portion of the sample using at least one of the one or more nitric oxide sensors in the first sensor line, and comparing the amount of nitric oxide detected in the first portion of the sample with a predetermined amount of nitric oxide to be delivered to the mammal. In some embodiments, a method can include injecting a calibration standard having a known amount of nitric oxide into a second sensor line including one or more nitric oxide sensors, detecting an amount of nitric oxide in the second sensor line, and comparing the amount of nitric oxide detected in the second sensor line with the known amount of nitric oxide in the calibration standard. In some embodiments, injecting a calibration sample into the second sensor line can occur while the first portion of the sample is being communicated into the first sensor line.

In some embodiments, injecting the calibration standard having the known amount of nitric oxide into a second sensor line can include injecting the calibration standard for a period of time. In some cases, a period of time can be less than 15 minutes, less than 5 minutes, less than 30 seconds, less than 10 seconds or less than 5 seconds.

In some embodiments, injecting the calibration standard having a known amount of nitric oxide into the second sensor line can include pulsing a calibration standard having a known amount of nitric oxide into the second sensor line from a calibration source at a first time. In some embodiments, detecting the amount of nitric oxide in the second sensor line occurs at a second time. In some cases, the difference between the first time and the second time can be the time period it takes for a pulse of the calibration standard to traverse from the calibration source to the at least one of the one or more nitric oxide sensors in the second sensor line.

In another aspect, a system for delivering nitric oxide can include a reservoir including two or more restrictors and a delivery platform. In some embodiments, each restrictor can include a first end and a second end. In some cases, a first end can be coupled to the reservoir and a second end can be coupled to a delivery platform. In some embodiments, a delivery platform can include at least one receptacle including a support and a reducing agent. In some embodiments, the system can include a patient interface.

In another aspect, a method of delivering nitric oxide can include releasing a nitric oxide-releasing compound from a reservoir into a delivery platform via two or more restrictors. In some embodiments, a delivery platform can include at least one receptacle including a support and a reducing agent. In some embodiments, the method can include communicating the nitric oxide-releasing compound through the at least one receptacle and contacting the nitric oxide-releasing compound with the reducing agent to generate nitric oxide. In some embodiments, the method can include delivering nitric oxide from an outlet of the delivery platform.

In another aspect, a sensor module can include a first sensor line including one or more nitric oxide sensors and a second sensor line including one or more nitric oxide sensors.

In some embodiments, the sensor module can include a controller. In some embodiments, the controller can be configured to receive a first detection signal from a first nitric oxide sensor in the first sensor line. In some embodiments, the controller can be configured to receive a second detection signal from a second nitric oxide sensor in the second sensor line. In some embodiments, the controller can be configured to compare the first detection signal to a nitric oxide-signal representing a predetermined amount of nitric oxide to be delivered via the delivery line. In some embodiments, the controller can be configured to compare the second detection signal to the nitric oxide-signal. In some embodiments, the controller can be configured to compare the first detection signal to the second detection signal.

In another aspect, a sensor module can include a first sensor line including one or more nitric oxide sensors, a first controller, where the controller can be configured to receive a first detection signal from a first nitric oxide sensor in the first sensor line, a second sensor line including one or more nitric oxide sensors, and a second controller, where the controller can be configured to receive a second detection signal from a second nitric oxide sensor in the second sensor line. In some embodiments, the first controller can be configured to compare the first detection to a nitric oxide-signal representing a predetermined amount of nitric oxide to be delivered via the delivery line. In some embodiments, the second controller can be configured to compare the second detection to the nitric oxide-signal.

In another aspect, a method of calibrating a nitric oxide delivery system can include detecting an amount of nitric oxide in a sample gas using a first sensor line including one or more nitric oxide sensors, and simultaneously calibrating a second sensor line including one or more nitric oxide sensors.

In another aspect, a method of calibrating a nitric oxide delivery system can include passing a sample gas including a first amount of nitric oxide through a first sensor line including a first nitric oxide sensor, pulsing a calibration gas including a known amount of nitric oxide into the first sensor line, detecting a total amount of nitric oxide in the first sensor line at a predetermined time after the calibration gas was in pulsed into the first sensor line using the first nitric oxide sensor, where the total amount of nitric oxide can include the first amount of nitric oxide and the second amount, generating a total signal representing the total amount of nitric oxide in the first sensor line at the predetermined time, subtracting the signal representing the first amount of nitric oxide from total signal to determine the signal representing the amount of nitric oxide detected from the calibration gas, calculating the amount of nitric oxide detected from the calibration gas based on the signal representing the amount of nitric oxide detected from the calibration gas, and comparing the amount of nitric oxide detected from the calibration gas with the known amount of nitric oxide in the calibration gas.

In another aspect, a method of delivering nitric oxide to a mammal can include communicating a first gas including nitric oxide and/or nitrogen dioxide through a dilution line. In some embodiments the first gas comprises nitric oxide and/or nitrogen dioxide in nitrogen. In some embodiments, a method can include diluting the first gas in an inert gas, for example nitrogen gas, to reduce the concentration of nitric oxide and/or nitrogen dioxide in the first gas, preferably to a predetermined amount. A predetermined amount can be less than 800 ppm, less than 500 ppm, less than 200 ppm, less than100 ppm, less than 50 ppm, less than 20 ppm, less than 10 ppm, less than 5 ppm, or less than 2 ppm. A predetermined amount can be more than 0.1 ppm, more than1 ppm, more than 5 ppm, more than 10 ppm, more than 20 ppm, more than 50 ppm, more than 100 ppm, more than 200 ppm, or more than 500 ppm. In embodiments where both nitric oxide and nitrogen dioxide are present in the first gas, the concentration of nitric oxide and nitrogen dioxide can be the value compared to the predetermined value. The concentration of nitric oxide and nitrogen dioxide can be determined by adding the concentration of nitric oxide to the concentration of nitrogen dioxide. In other embodiments where both nitric oxide and nitrogen dioxide are present in the first gas, the concentration of nitric oxide or the concentration of nitrogen dioxide can be compared to the predetermined value. In some embodiments, the diluting can occur in the dilution line. In some embodiments, a dilution line can include a dilution chamber.

For example, a source of 800 ppm of nitric oxide in nitrogen gas can be diluted to 50 ppm of nitric oxide in nitrogen gas. The 50 ppm gas can then be used and diluted within a platform to deliver 5 ppm of nitric oxide to a mammal.

In some embodiment, a method can include communicating a first gas including nitric oxide through a dilution line to a first platform. In other words, diluting the first gas in an inert gas, for example nitrogen gas, to reduce the concentration of nitric oxide in the first gas, preferably to a predetermined amount, can occur before the first gas is communicated to a first platform. In some embodiments, a dilution line is coupled to a first platform. A first platform can include a first receptacle including a first support and a first reducing agent. In some cases, a method can include communicating a first gas including nitric oxide through a first platform. Communicating a first gas including nitric oxide through a first platform can include contacting the first gas with the first reducing agent to generate nitric oxide.

In some embodiments, a method of delivering nitric oxide to a mammal can include communicating a second gas including nitric oxide and/or nitrogen dioxide through a dilution line. In some embodiments the second gas comprises nitric oxide and/or nitrogen dioxide in nitrogen. In some embodiments, a method can include diluting the second gas in an inert gas, for example nitrogen gas, to reduce the concentration of nitric oxide and/or nitrogen dioxide in the second gas, preferably to a predetermined amount. A predetermined amount can be less than 800 ppm, less than 500 ppm, less than 200 ppm, less than100 ppm, less than 50 ppm, less than 20 ppm, less than 10 ppm, less than 5 ppm, or less than 2 ppm. A predetermined amount can be more than 0.1 ppm, more than1 ppm, more than 5 ppm, more than 10 ppm, more than 20 ppm, more than 50 ppm, more than 100 ppm, more than 200 ppm, or more than 500 ppm. In embodiments where both nitric oxide and nitrogen dioxide are present in the second gas, the concentration of nitric oxide and nitrogen dioxide can be the value compared to the predetermined value. The concentration of nitric oxide and nitrogen dioxide can be determined by adding the concentration of nitric oxide to the concentration of nitrogen dioxide. In other embodiments where both nitric oxide and nitrogen dioxide are present in the second gas, the concentration of nitric oxide or the concentration of nitrogen dioxide can be compared to the predetermined value. In some embodiments, the diluting can occur in the dilution line. In some embodiments, a dilution line can include a dilution chamber.

In some embodiment, a method can include communicating a second gas including nitric oxide through a dilution line to a second platform. In other words, diluting the second gas in an inert gas, for example nitrogen gas, to reduce the concentration of nitric oxide in the second gas, preferably to a predetermined amount, can occur before the second gas is communicated to a second platform. In some embodiments, a dilution line is coupled to a second platform. A second platform can include a second receptacle including a second support and a second reducing agent. In some cases, a method can include communicating a second gas including nitric oxide through a second platform. Communicating a second gas including nitric oxide through a second platform can include contacting the second gas with the second reducing agent to generate nitric oxide.

Other features, objects, and advantages will be apparent from the description, drawings, and claims.

### BRIEF DESCRIPTION OF THE DRAWINGS

FIG. 1 is a schematic of a receptacle.
FIG. 2 is a schematic of a reservoir including two restrictors.
FIG. 3 is a schematic of a restrictor.
FIG. 4 is an illustration of a reservoir including one restrictor.
FIG. 5 is an illustration of a reservoir including one restrictor.
FIG. 6 is a schematic of a dual platform system.
FIG. 7 is a schematic of a dual platform system.
FIG. 8 is a schematic of a platform of a dual platform system.

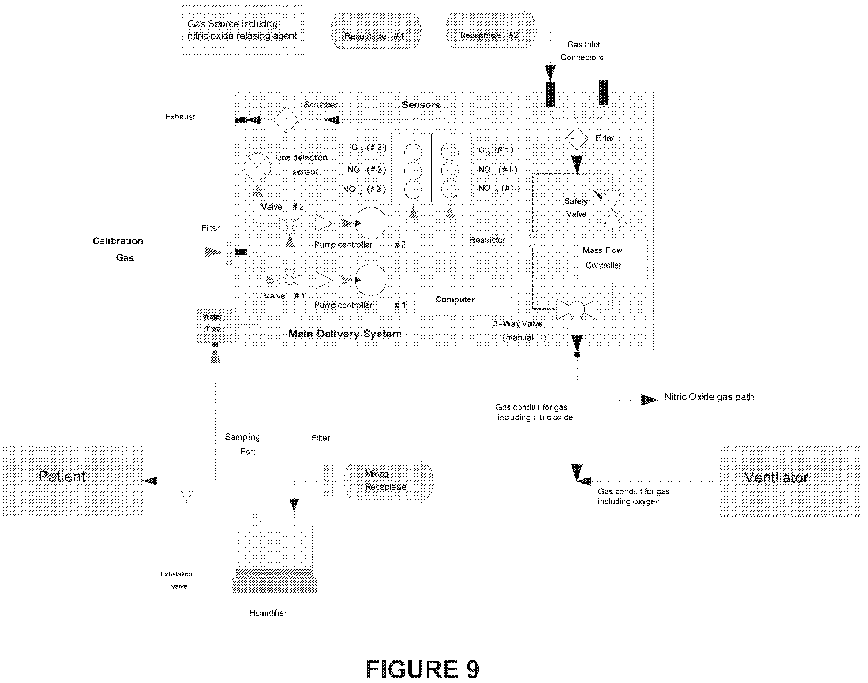

FIG. 9 is a schematic of a platform.

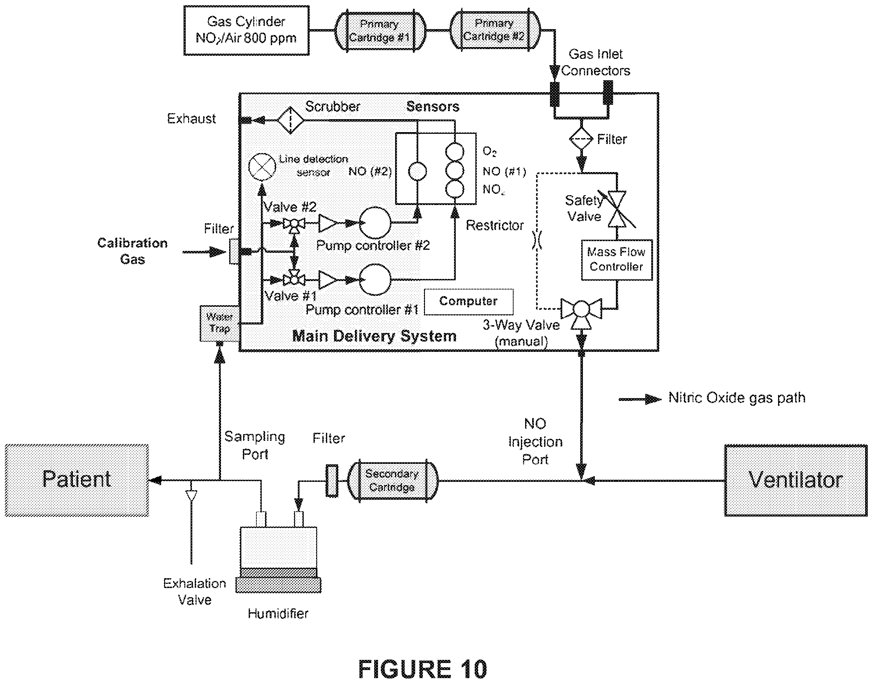

FIG. 10 is a schematic of a platform.
FIG. 11 is a schematic of a sensor module with calibration standards.
FIG. 12 is a schematic of a sensor module with calibration standards.
FIG. 13 is a box diagram of a dual platform system.

### DETAILED DESCRIPTION

Nitric oxide, also known as nitrosyl radical, is a free radical that is an important signaling molecule in pulmonary vessels. Nitric oxide can moderate pulmonary hypertension caused by elevation of the pulmonary arterial pressure. Inhaling low concentrations of nitric oxide, for example, in the range of 0.01-100 ppm can rapidly and safely decrease pulmonary hypertension in a mammal by vasodilation of pulmonary vessels.

Some disorders or physiological conditions can be mediated by inhalation of nitric oxide. The use of low concentrations of inhaled nitric oxide can prevent, reverse, or limit the progression of disorders which can include, but are not limited to, acute pulmonary vasoconstriction, traumatic injury, aspiration or inhalation injury, fat embolism in the lung, acidosis, inflammation of the lung, adult respiratory distress syndrome, acute pulmonary edema, acute mountain sickness, post cardiac surgery acute pulmonary hypertension, persistent pulmonary hypertension of a newborn, perinatal aspiration syndrome, haline membrane disease, acute pulmonary thromboembolism, heparin-protamine reactions, sepsis, asthma and status asthmaticus or hypoxia. Nitric oxide can also be used to treat chronic pulmonary hypertension, bronchopulmonary dysplasia, chronic pulmonary thromboembolism and idiopathic or primary pulmonary hypertension or chronic hypoxia. Advantageously, nitric oxide can be generated and delivered in the absence of harmful side products, such as nitrogen dioxide. The nitric oxide can be generated at a concentration suitable for delivery to a mammal in need of treatment.

When delivering nitric oxide (NO) for therapeutic use to a mammal, it can be important to avoid delivery of nitrogen dioxide (NO₂) to the mammal. Nitrogen dioxide (NO₂) can be formed by the oxidation of nitric oxide (NO) with oxygen (O₂). The rate of formation of nitrogen dioxide (NO₂) can be proportional to the oxygen (O₂) concentration multiplied by the square of the nitric oxide (NO) concentration. A NO delivery system can convert nitrogen dioxide (NO₂) to nitric oxide (NO). Additionally, nitric oxide can form nitrogen dioxide at increased concentrations.

The reliability of a nitric oxide delivery system can be absolutely critical to the survival of the patient. While an overdose of up about 80 ppm of NO can be tolerated for a short time, abrupt or sudden removal of the NO can prove fatal. This can be especially true for neonates (and other patients who have become dependent upon the drug) due to a "rebound" effect. In fact, the FDA MAUDE data base cites several instances of patient death due to an equipment failure. For this reason, it can be essential to design and build a delivery system with 100% redundancy so that a failure of the system could only occur if more than one fault occurred at the same time, in two redundant, independent platforms. This design concept can greatly reduce the likelihood of a catastrophic failure. A key feature of the dual platform system is that both are operated in parallel, so that one may take the full load if the other fails. This approach can be considered similar to that of a twin engine aircraft, where the plane can still fly, even if one engine fails on takeoff or landing.

The dual system can be designed to function similar to a single platform, but can have the added benefit of being redundant. A dual platform system can be essentially two completely independent nitric oxide inhalation platform systems, each which can stand by itself and supply the patient's complete needs for NO inhalation gas. Because there are two independent platforms, the parallel platform can take over in the event of a failure. The FDA has required nitric oxide delivery devices to have independent backup systems to provide NO inhalation gas in the event of failure of the primary device. Having a dual platform system can simplify the engineering of each independent platform because each platform can qualify as the backup system for the other. Thus, an external back-up system would not be required because the dual system can be defined as internally encompassing a primary platform and a back-up platform. For a failure of the dual platform system to occur, a failure must occur in both platforms at the same time. While theoretically possible, the odds of a two failures occurring at the same time in two independent platforms is extremely remote.

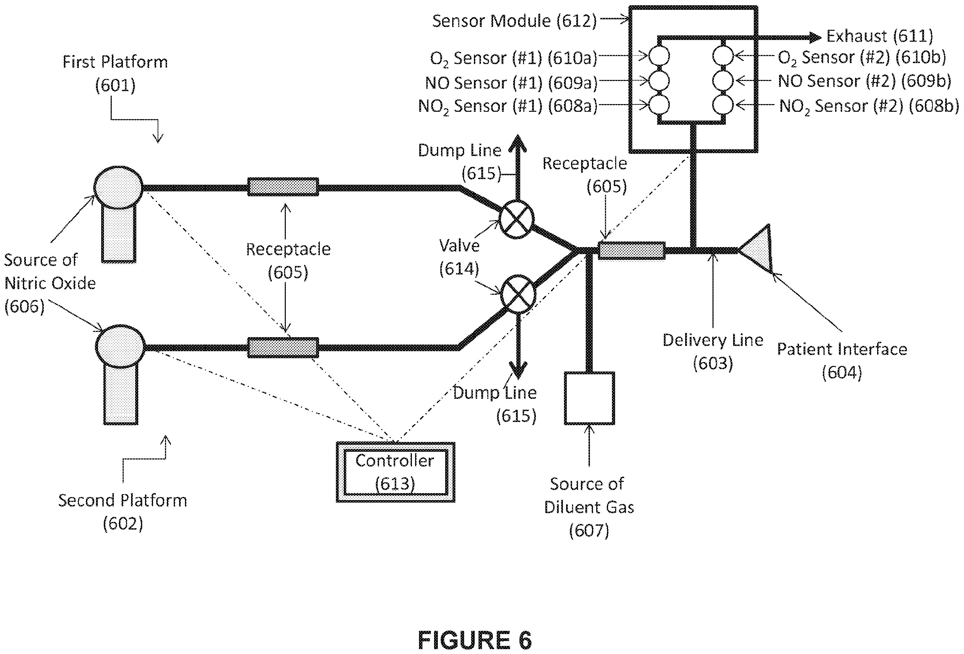

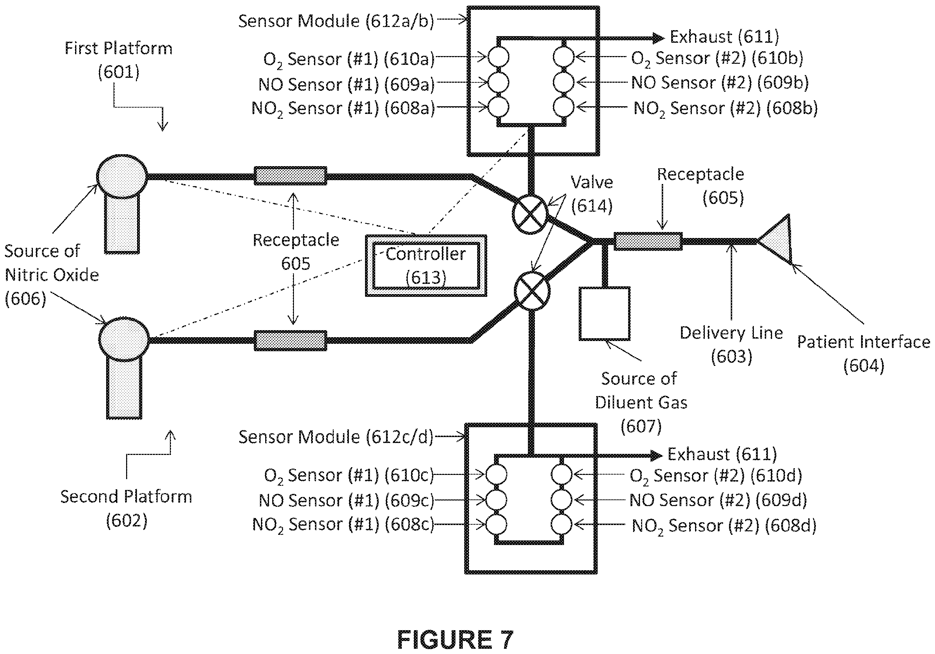

Schematics of exemplary dual platform systems are shown in Figures 6 and 7. Generally, as shown in Figures 6 and 7, each platform 601, 602 can include a source of nitric oxide 606. The source of nitric oxide 606 can release a gas including a nitric oxide-releasing compound into its corresponding platform 601, 602. The platform 601, 602 can communicate the gas through a receptacle 605 included in the platform 601, 602. A receptacle 605 can be capable of converting a nitric oxide-releasing compound to nitric oxide. As a result, once a gas including a nitric oxide-releasing compound passes through a receptacle 605, the gas can include nitric oxide. Preferably, all of the nitric oxide-releasing compound in the gas can be converted to nitric oxide by a receptacle 605. In addition to nitric oxide-releasing agent, a receptacle 605 can convert any nitrogen dioxide that forms within a platform 601, 602 to nitric oxide. Each platform can also include at least one valve 614 that has a variety of positions. The position of the valve 614 can determine the flow path of gas through a platform 601, 602, and consequently, the dual platform system. At least one valve 614 can be within a platform 601, 602 and/or between a platform 601, 602 and a delivery line 603. Both platforms 601, 602 can be coupled to one end of a delivery line 603. At the other end of the delivery line 603, the delivery line 603 can include a patient interface 604. A patient interface 604 can be adapted to deliver the gas including nitric oxide to a mammal, preferably a mammal's mouth and/or nose.

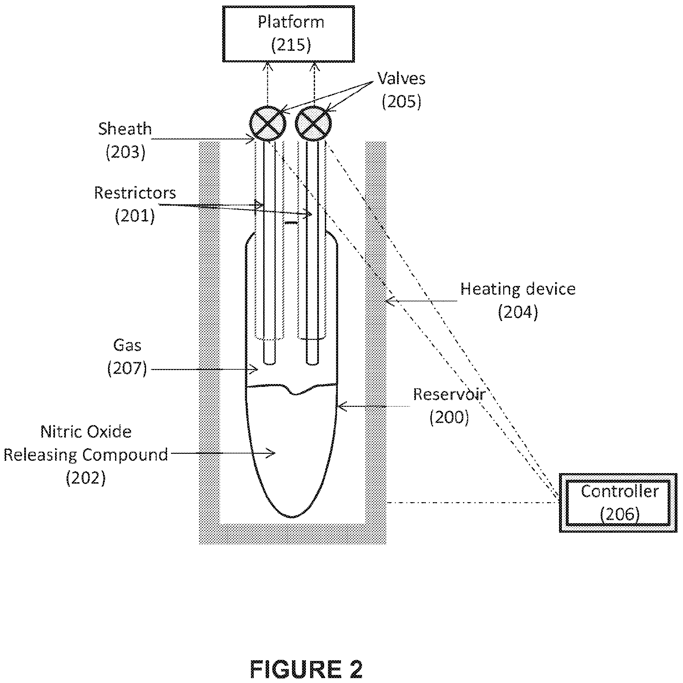

More specifically, a platform 601, 602 can include a source of nitric oxide 606. A source of nitric oxide can include a gas bottle, for example, a pressurized gas bottle or a gas tank. Referring to Figure 2, a source of nitric oxide can also include a reservoir 200. As shown in Figure 2, in an exemplary embodiment, a reservoir 200 can include one or more restrictors 201. The reservoir can also include a nitric oxide-releasing compound 202. The reservoir 200 can operate at a temperature above room temperature. To get the temperature of the reservoir up to the operating temperature, a heating device 204 can be utilized. The heating device 204 can also be used to regulate the temperature of the one or more restrictors 201. At an increased temperature, the nitric oxide-releasing compound in the reservoir can be vaporized into nitric oxide-releasing compound in the form of a gas 207. To be clear, gas 207 includes nitric oxide-releasing compound 202. The vapor pressure of the gas 207 within the reservoir 200 can cause the gas 207 to enter and traverse the one or more restrictors 201. Because the vapor pressure in the reservoir 200 and the one or more restrictors 201 can be relatively high, a sheath 203 can surround each restrictor 201. Each restrictor can include a valve 205, which can regulate whether or not the gas 207 can pass out of the restrictor 201 and into a platform 215. Each valve 205 and the heating device 204 can be controlled by a controller 206.

A reservoir 200 can be any compartment or portion of a compartment suitable for holding a nitric oxide-releasing compound 202. A nitric oxide-releasing compound 202 can include N₂O₄, NO₂ or NO, or other compounds which can generate N₂O₄, NO₂ or NO, as discussed further herein. The reservoir 200 can hold a liquid or a solid, but preferably the reservoir 200 can hold liquid N₂O₄. The reservoir 200 can be made of any material, which does not react with or adsorb N₂O₄, NO₂ or NO, or other compounds which can generate N₂O₄, NO₂ or NO. The material should also be able to tolerate heat within the appropriate range, discussed herein, and repeated heating and cooling.

The amount of nitric oxide-releasing compound 202 in the reservoir 200 can be less than about 5.0 g, less than about 2.0 g, less than about 1.0 g, less than about 0.50 g, less than 0.25 g or less than 0.10 g; the amount of nitric oxide-releasing compound 202 in the reservoir 200 can be greater than about 0.05 g, greater than about 0.10 g, greater than about 0.20 g, greater than about 0.50 g or greater than about 1.0 g. The amount of nitric oxide-releasing compound 202 in the reservoir 200 can be less than about 5 ml, less than about 2 ml, less than about 1 ml, less than about 0.5 ml, less than about 0.25 ml or less than about 0.10 ml; amount of nitric oxide-releasing compound 202 in the reservoir 200 can be greater than about 0.001 ml, greater than about 0.01 ml, greater than about 0.05, greater than about 0.10 ml, greater than about 0.25ml, greater than about 0.50 ml or greater than about 1.0 ml.

In one exemplary embodiment, nitric oxide-releasing compound 202 can be stored in a small, pressurized reservoir. If the nitric oxide-releasing compound 202 is N₂O₄, for a delivery concentration of 80 parts per million in 1 liter of air per minute, for example, the amount of N₂O₄ needed for a 24 hour supply can be approximately 0.24 g, or 0.15 ml. N₂O₄ boils at 21 °C, so a reservoir can be heated to above this temperature in order to achieve a vapor pressure of NO₂ that is greater than atmospheric pressure.

A reservoir 200 can also include gas 207 in a space over the nitric oxide-releasing compound. The gas can include nitric oxide and/or nitrogen dioxide.

As seen in Figure 2, a reservoir 200 can include one or more restrictors 201 (e.g. 1, 2, 3, 4 or 5). A restrictor 201 can be any device which can limit the flow of NO₂ from the reservoir 200. A restrictor 201 can require that there be enough vapor pressure to force the NO₂ vapor out of the reservoir 200 and into the restrictor 201.

In some cases, the restrictor 201 can be an orifice (not shown). The restrictor 201 can be coupled to the reservoir 200. For example, the restrictor 201 can include a tube, most preferably, a capillary tube. The capillary tube can be a quartz capillary tube. The capillary tube can be a narrow bore capillary tube, which can allow for simple, reproducible and accurate use, as well as a cost effective solution. A convenient commercially available restrictor 201 can be a narrow bore quartz capillary tube that can be used for gas chromatography (GC).

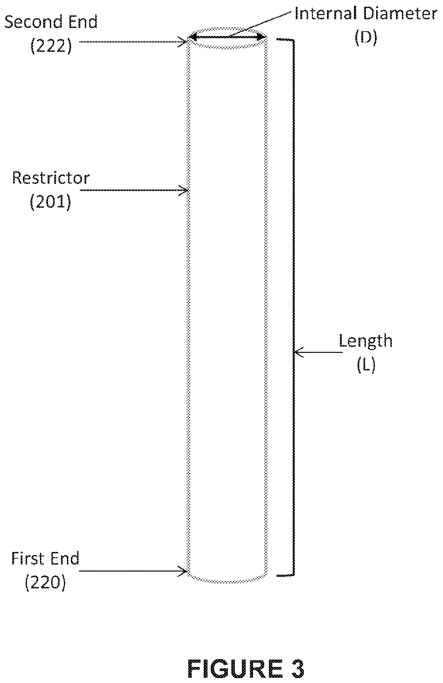

Referring to Figure 3, a restrictor 201 can include a first end 220 and a second end 222. In some embodiments, the first end 220 of the restrictor 201 can be coupled to a reservoir 200 and the second end 222 can be coupled to a platform. In some cases, the second end 222 of a restrictor 201 can be initially sealed or closed. The second end 222, which was previously sealed or closed, can be opened, unsealed or include a broken seal, which can allow for a nitric oxide-releasing agent 202 to pass through the restrictor 201 and into a platform.

As shown in Figure 3, a restrictor 201 can have a length (L) corresponding to the distance between the first end 220 and the second end 222. In some embodiments, the length (L) of the restrictor 201 can be at least about 0.1 inch, at least about 0.25 inch or at least about 0.5 inch; the length can be at most about 4 inches, at most about 2 inches, at most about 1 inch, or at most about 0.5 inch. Preferably, the restrictor 201 can have a length (L) of about 0.75 inch.

As also shown in Figure 3, a restrictor 201 can have an internal diameter (D). The internal diameter (D) of the restrictor 201 can be at least about 1 micron, at least about 5 microns or at least about 10 microns; the internal diameter (D) can be at most about 100 microns, at most about 50 microns, at most about 25 microns, or at most about 10 microns. Preferably, the restrictor 201 can have a diameter (D) of about 10 microns.

In a preferred embodiment, one restrictor has dimensions (i.e. L and D) that allow greater than 50%, greater than 60%, greater than 70%, greater than 80% or greater than 90% of the total amount of nitric oxide-releasing compound to pass through that restrictor. An additional one or more restrictors can have dimensions (i.e. L and D) that allow the remainder of the nitric oxide-releasing compound to pass through the additional one or more restrictors. This configuration can provide a more precise way to deliver a concentration of nitric oxide-releasing agent or nitric oxide. One restrictor can be utilized for gross control over the amount of nitric oxide-releasing compound released from the reservoir, while one or more additional restrictors can be utilized for fine control over the amount of nitric oxide-releasing compound released from the reservoir.

The amount of nitric oxide-releasing compound that is forced out of the reservoir at any temperature can be dependent upon the internal diameter (D) of the restrictor 201and the length (L) of the restrictor 201. Thus, the two key design variables can be: 1) the temperature at which the reservoir (including restrictors) operate, and 2) the diameter (D) and length (L) of the restrictor 201. For example, at about 45 °C, a tube with an internal diameter of 10 microns and a length of 0.75 inches can be used to provide 80 ppm of NO₂ in an air stream of 11/min.

A restrictor 201 can be made of materials known to those of skill in the art. The material should not react with or adsorb N₂O₄, NO₂ or NO, or other compounds which can generate N₂O₄, NO₂ or NO. The material should also be able to tolerate heat within the appropriate range, discussed herein, and repeated heating and cooling.

Referring to Figure 2, a heating device 204 can be thermally connected with the reservoir 200 and/or the one or more restrictors 201. While the heating device 204 is referred to as a "heating" device, it should be understood that the heating device 204 can be used to increase or decrease the temperature of the reservoir 200 and/or the one or more restrictors 201. The heating device 204 can also be used to maintain the temperature of the reservoir 200 and/or the one or more restrictors 201.

The heating device 204 can bring the temperature of the reservoir 200 and/or the one or more restrictors 201 to a predetermined temperature that can be set in advance by a user. The predetermined temperature can be entered into a controller 206. Additionally, the heating device 204 can bring the temperature of the reservoir 200 and/or the one or more restrictors 201 to a predetermined temperature that is calculated by a controller 206. For example, the temperature at which the reservoir 200 is operating can be determined based on factors, such as, the number of restrictors, the length of the restrictor(s), the internal diameter of the restrictor(s), the desired concentration of nitric oxide-releasing compound in a gas in a platform, and the amount of nitric oxide-releasing compound in the reservoir. As the amount of nitric oxide-releasing compound 202 in the reservoir decreases, it may be necessary to increase the temperature at which the reservoir 200 should operate. Accordingly, the predetermined temperature calculated by the controller 206 would increase and cause the heating device 204 to increase the temperature of the reservoir 200 and/or the one or more restrictors 201. As the temperature increased, the amount of nitric oxide-releasing agent leaving the reservoir 200 through the restrictor 201 can also increase. Any increase in temperature can be gradual or incremental.

Because the pressure inside a reservoir 200 and/or the one or more restrictors 201 can be high, it can be beneficial to include a sheath 203 around each restrictor 201 to prevent leaking or breakage of a restrictor 201. A sheath can be made of any durable material, for example, a metal.

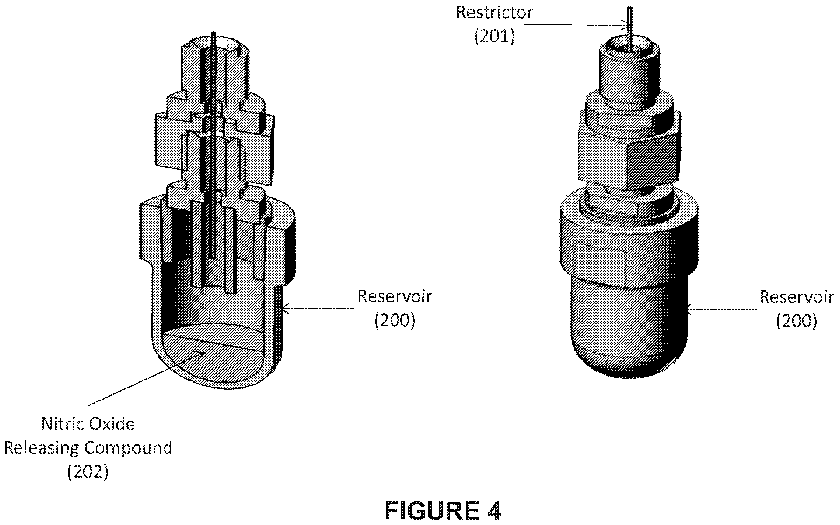

An alternate embodiment of a device including a reservoir and one restrictor is shown in Figure 4. It should be understood that the embodiment in Figure 4 can be modified to include more than one restrictor.

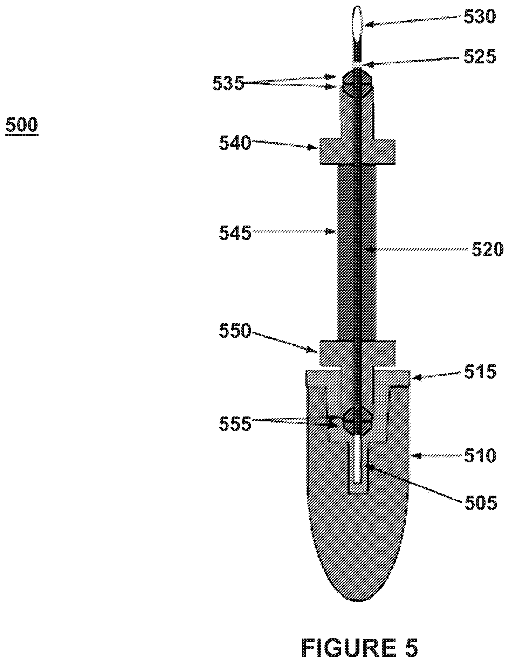

An alternate embodiment of a device including a reservoir and a restrictor is shown in Figure 5. A restrictor can be a capillary tube 520, which can be about 1-inch x 10 um internal diameter (TSP010375 Flexible Fused Silica Capillary Tubing Polymicro Technologies). The capillary tube 520 can be inserted through a metal sheath (303 S.S.) 545 made up of two GC nuts 540 and 550 (1/16" Stainless Steel Nut Valco P/N ZN1-10) connected via their tops to the metal sheath 545. Two graphite ferrules 555 (Graphite Ferrules P/N 20227 1/16" X 0.4mm Restek) with their flat ends touching can be placed on one end of the capillary tube 520, which has the polyamide coating 505 removed below the graphite ferrules 555 (e.g., by burning off the polyamide with a flame). The graphite ferrules 555 can hold the capillary tube 520 securely when the nut 540 is inserted into a separate female end of an adaptor 515, which can be itself inserted into the metal (303 S.S.) reservoir container 510. The adaptor 515 can have a metal sheath 545 on the reservoir end that can cover and protect the area of the capillary without polyamide. It should be understood that the embodiment in Figure 5 can be modified to include more than one restrictor.

Referring back to Figures 2 and 3, a restrictor 201 can include a valve 205. Typically, the valve 205 can be located close to the first end 220 or the second end 222. The valve 205 can control whether the nitric oxide-releasing compound 202 can pass from the reservoir 200 through the restrictor 201 and/or whether the nitric oxide-releasing compound 202 can pass from the restrictor 201 into the platform. A valve 205 can have an open position that allows a fluid, preferably a gas, to pass through the restrictor 201 and a closed position that prevents a fluid, preferably a gas, from passing through the restrictor 201. A valve 205 can have a plurality of positions between the open position and the closed position that allow a fraction of the full amount of fluid that can pass through the restrictor (e.g. when it is in the open position) to pass through the restrictor 201. A controller 206 can switch the position of the valve 205 on each restrictor.

Once the gas including the nitric oxide-releasing compound is released from a nitric oxide source, the gas including the nitric oxide-releasing compound can enter into the platform. The gas including the nitric oxide-releasing compound can then be communicated through the platform. As shown in Figure 6, a platform can include one or more receptacles 605. A receptacle 605 can convert the nitric oxide-releasing compound in the platform to nitric oxide (NO). As the nitric oxide-releasing compound is converted to nitric oxide, the concentration of nitric oxide-releasing compound in the gas will decrease, while the concentration of nitric oxide in the gas will increase. Preferably all of the nitric oxide-releasing compound will be converted to nitric oxide.

A nitric oxide-releasing compound can include one or more of nitrogen dioxide (NO₂), dinitrogen tetroxide (N₂O₄) or nitrite ions (NO₂⁻). Nitrite ions can be introduced in the form of a nitrite salt, such as sodium nitrite. A nitric oxide-releasing compound can also include a compound having a N₂O₂- functional group.

A receptacle 605 can include a reducing agent or a combination of reducing agents. A number of reducing agents can be used depending on the activities and properties as determined by a person of skill in the art. In some embodiments, a reducing agent can include a hydroquinone, glutathione, and/or one or more reduced metal salts such as Fe(II), Mo(VI), NaI, Ti(III) or Cr(III), thiols, or NO₂⁻. A reducing agent can include 3,4 dihydroxy-cyclobutene-dione, maleic acid, croconic acid, dihydroxy-fumaric acid, tetra-hydroxy-quinone, p-toluene-sulfonic acid, tricholor-acetic acid, mandelic acid, 2-fluoro-mandelic acid, or 2, 3, 5, 6-tetrafluoro-mandelic acid. A reducing agent can be an antioxidant. An antioxidant can include any number of common antioxidants, including ascorbic acid, alpha tocopherol, and/or gamma tocopherol. A reducing agent can include a salt, ester, anhydride, crystalline form, or amorphous form of any of the reducing agents listed above. A reducing agent can be used dry or wet. For example, a reducing agent can be in solution. A reducing agent can be at different concentrations in a solution. Solutions of the reducing agent can be saturated or unsaturated. While a reducing agent in organic solutions can be used, a reducing agent in an aqueous solution is preferred. A solution including a reducing agent and an alcohol (e.g. methanol, ethanol, propanol, isopropanol, etc.) can also be used.

A receptacle 605 can include a support. A support can be any material that has at least one solid or non-fluid surface (e.g. a gel). It can be advantageous to have a support that has at least one surface with a large surface area. In preferred embodiments, the support can be porous. One example of a support can be surface-active material, for example, a material with a large surface area that is capable of retaining water or absorbing moisture. Specific examples of surface active materials can include silica gel or cotton.

A support can include a reducing agent. Said another way, a reducing agent can be part of a support. For example, a reducing agent can be present on a surface of a support. One way this can be achieved can be to coat a support, at least in part, with a reducing agent. In some cases, a support can be coated with a solution including a reducing agent. Preferably, a support can employ a surface-active material coated with an aqueous solution of antioxidant as a simple and effective mechanism for making the conversion. Generation of NO from a nitric oxide-releasing compound performed using a support with a reducing agent can be the most effective method, but a reducing agent alone can also be used to convert nitric oxide-releasing compound to NO.

In some circumstances, a support can be a matrix or a polymer, more specifically, a hydrophilic polymer. A support can be mixed with a solution of the reducing agent. The solution of reducing agent can be stirred and strained with the support and then drained. The moist support-reducing agent mixture can be dried to obtain the proper level of moisture. Following drying, the support-reducing agent mixture may still be moist or may be dried completely. Drying can occur using a heating device, for example, an oven or autoclave, or can occur by air drying.

In general, a nitric oxide-releasing compound can be converted to NO by bringing a gas including the nitric oxide-releasing compound in contact with a reducing agent. In one example, a gas including a nitric oxide-releasing compound can be passed over or through a support including a reducing agent. When the reducing agent is ascorbic acid (i.e. vitamin C), the conversion of nitrogen dioxide to nitric oxide can be quantitative at ambient temperatures.

The generated nitric oxide can be delivered to a mammal, which can be a human. To facilitate delivery of the nitric oxide, a system can include a patient interface. Examples of a patient interface can include a mouth piece, nasal cannula, face mask, fully-sealed face mask or an endotracheal tube. A patient interface can be coupled to a delivery conduit. A delivery conduit can include a ventilator or an anesthesia machine.

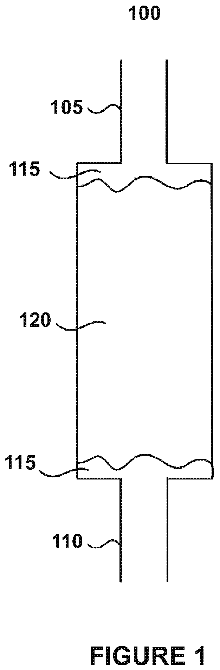

Fig. 1 illustrates an exemplary embodiment of a receptacle for generating NO by converting a nitric oxide-releasing compound to NO. An example of a receptacle can be a cartridge. A cartridge can be inserted into and removed from an apparatus, platform or system. Preferably, a cartridge is replaceable in the apparatus, platform or system, and more preferably, a cartridge can be disposable.

The receptacle 100 can include an inlet 105 and an outlet 110. Screen and glass wool 115 can be located at either or both of the inlet 105 and the outlet 110. The remainder of the receptacle 100 can include a support 120. In a preferred embodiment, a receptacle 100 can be filled with a surface-active material. The surface-active material can be soaked with a saturated solution of antioxidant in water to coat the surface-active material. The screen and glass wool 115 can also be soaked with the saturated solution of antioxidant in water before being inserted into the receptacle 100.

In general, a process for converting a nitric oxide-releasing compound to NO can include passing a gas including a nitric oxide-releasing compound into the inlet 105. The gas can be communicated to the outlet 110 and into contact with a reducing agent. In a preferred embodiment, the gas can be fluidly communicated to the outlet 110 through the surface-active material 120 coated with a reducing agent. As long as the surface-active material remains moist and the reducing agent has not been used up in the conversion, the general process can be effective at converting a nitric oxide-releasing compound to NO at ambient temperature.

The inlet 105 may receive the gas including a nitric oxide-releasing compound from a nitric oxide source. A nitric oxide-releasing compound can be mixed in another gas, for example, nitrogen (N₂), air, or oxygen (O₂). A wide variety of flow rates and NO₂ concentrations have been successfully tested through a receptacle, ranging from only a few ml per minute to flow rates of up to 5,000 ml per minute.

The conversion of a nitric oxide-releasing compound to NO can occur over a wide range of concentrations of a nitric oxide-releasing compound. For example, experiments have been carried out at concentrations in air of from about 2 ppm NO₂ to 100 ppm NO₂, and even to over 1000 ppm NO₂. In one example, a receptacle that was approximately 6 inches long and had a diameter of 1.5-inches was packed with silica gel that had first been soaked in a saturated aqueous solution of ascorbic acid. The moist silica gel was prepared using ascorbic acid designated as A.C.S reagent grade 99.1 % pure from Aldrich Chemical Company and silica gel from Fischer Scientific International, Inc., designated as S8 32-1, 40 of Grade of 35 to 70 sized mesh. Other sizes of silica gel can also be effective. For example, silica gel having an eighth-inch diameter can also work.

In another example, silica gel was moistened with a saturated solution of ascorbic acid that had been prepared by mixing 35% by weight ascorbic acid in water, stirring, and straining the water/ascorbic acid mixture through the silica gel, followed by draining. The conversion of NO₂ to NO can proceed well when the support including the reducing agent, for example, silica gel coated with ascorbic acid, is moist. In a specific example, a receptacle filled with the wet silica gel/ascorbic acid was able to convert 1000 ppm of NO₂ in air to NO at a flow rate of 150 ml per minute, quantitatively, non-stop for over 12 days.

A receptacle 605 in a platform 601, 602 or a delivery line 603 may be used to supplement or replace some or all of the safety devices traditionally used during delivery of NO. For example, one type of safety device can warn of the presence of NO₂ in a gas when the concentration of NO₂ exceeds a preset or predetermined limit, usually 1 part per million or greater of NO₂. Such a safety device may be unnecessary when a receptacle is positioned in a delivery line just prior to the patient breathing the NO laden gas. A receptacle can convert any NO₂ to NO just prior to the patient breathing the NO laden gas, making a device to warn of the presence of NO₂ in gas unnecessary.

Furthermore, a receptacle placed near the outlet or exit of a delivery line can also reduce or eliminate problems associated with formation of NO₂ that occur due to transit times in the equipment, lines or tubing. As such, use of a receptacle can reduce or eliminate the need to ensure the rapid transit of the gas through the gas plumbing lines that may be needed in conventional applications.

In some cases, a receptacle can include heat-activated alumina. A receptacle with heat-activated alumina, such as supplied by Fisher Scientific International, Inc., designated as ASOS-212, of 8-14 sized mesh can be effective at removing low levels of NO₂ from an air or oxygen stream, and yet, can allow NO gas to pass through without loss. Activated alumina, and other high surface area materials like it, can be used to scrub NO₂ from a delivery line.

One advantage of using one or more receptacles in the platform can be that nitrogen dioxide (gaseous or liquid) or dinitrogen tetroxide can be used as the source of the NO. When nitrogen dioxide or dinitrogen tetroxide is used as a source for generation of NO, there may be no need for a pressurized gas bottle to provide NO gas to a platform. By eliminating the need for a pressurized gas bottle to provide NO, the platform may be simplified as compared with a conventional apparatus that is used to deliver NO gas to a patient from a pressurized gas bottle of NO gas. A NO delivery system that does not use pressurized gas bottles may be more portable than conventional systems that rely on pressurized gas bottles.

Because the generation of nitric oxide from nitric oxide-releasing compounds can be efficient and complete, the amount of nitric oxide-releasing compound in a gas can be approximately equivalent to the amount of nitric oxide to be delivered to a patient. For example, if a therapeutic dose of 20 ppm of nitric oxide is to be delivered to a patient, a gas including 20 ppm of a nitric oxide-releasing compound (e.g., NO₂) can be released from a nitric oxide source. The gas including 20 ppm of a nitric oxide-releasing compound can be passed through one or more receptacles to completely convert the 20 ppm of nitric oxide-releasing compound to 20 ppm of nitric oxide for delivery to the patient.

More typically in the dual platform system, the efficiency of conversion by receptacles can also allow for the amount of nitric oxide-releasing compound in a gas to be greater than the amount of nitric oxide to be delivered to a patient. For example, a gas including 800 ppm of a nitric oxide-releasing compound can be released from a nitric oxide source. The gas including 800 ppm of a nitric oxide-releasing compound can be passed through one or more receptacles to convert the 800 ppm of nitric oxide-releasing compound to 800 ppm of nitric oxide. The gas including 800 ppm of nitric oxide can then be diluted in a gas including oxygen (e.g., air) to obtain a gas mixture with 20 ppm of nitric oxide for delivery to a patient.

After a first gas including a nitric oxide-releasing compound has passed through a receptacle 605, the first gas can include nitric oxide. The first gas including nitric oxide can be communicated through the first platform 601 and into the delivery line 603. At the point where the first platform 601 is coupled to the delivery line 603, the first gas can include a first amount nitric oxide. Likewise, a second gas can be communicated as described above through the second platform 602 and into the delivery line 603. At the point where the second platform 602 is coupled to the delivery line 603, the second gas can include a second amount nitric oxide. Because a first platform can communicate a first gas at the same time as a second platform can communicate a second gas, a delivery gas in the delivery line 603 can include the first gas and the second gas. This can mean that the delivery gas includes a total amount of nitric oxide equivalent to the first amount of nitric oxide plus the second amount of nitric oxide. The first amount of nitric oxide can be greater than the second amount of nitric oxide. In preferred embodiments, the first amount of nitric oxide is significantly greater (e.g. at least four times greater or at least nine times greater) than the second amount of nitric oxide. By using two platforms that deliver different amounts of nitric oxide, finer control over the amount of nitric oxide delivered can be achieved. For example, the first platform can deliver a greater amount of nitric oxide and can be used for gross control of the total amount of nitric oxide delivered. The second can deliver a lesser amount of nitric oxide and can be used for fine control of the total amount of nitric oxide delivered. A total amount of nitric oxide in the delivery gas can be at most 100 ppm, at most 80 ppm, at most 60 ppm, at most 40 ppm, at most 20 ppm, at most 10 ppm, at most 5 ppm or at most 1 ppm.

The delivery gas in the delivery line 603 can also include a diluent gas. A source of diluent gas 607 can be coupled to the delivery line 603 and supply the diluent gas into the delivery line 603. A relatively high percentage of the delivery gas can be diluent gas. A diluent gas can include oxygen, for example, air.

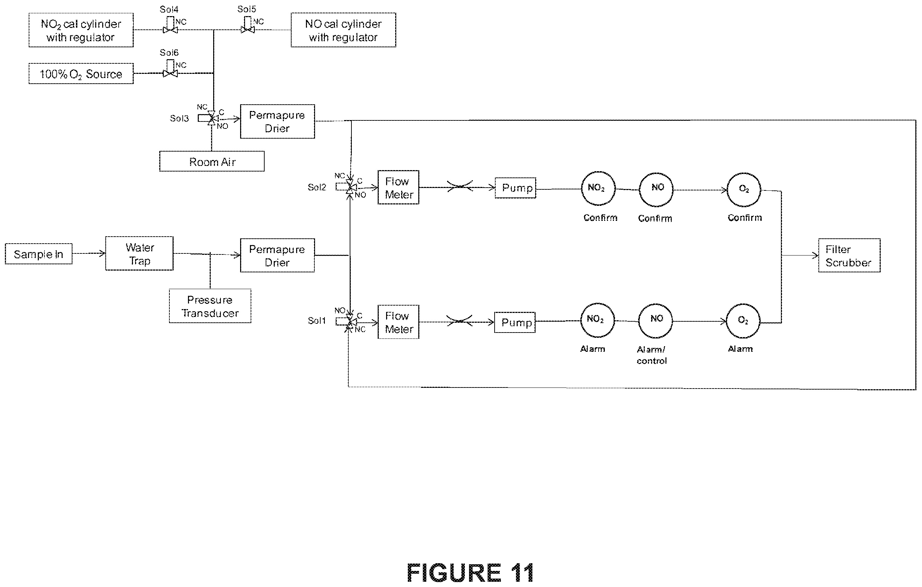

A dual platform system can include a sensor module 612, preferably coupled to delivery line 603. A sensor module 612 can include at least two sensor lines (a, b). Both the first (a) and second (b) sensors lines can include at least one nitric oxide sensor 609a, 609b. In some cases, the first (a) and second (b) sensor lines can include at least one nitrogen dioxide sensor 608a, 608b and/or at least one oxygen sensor 610a, 610b. A sample of the delivery gas can be taken from the delivery line 603. A first portion of the sample can be directed through the first sensor line (a). A second portion of the sample can be directed through the second sensor line (b). The first sensor line (a) can act as an alarm sensor line and detect the concentration of the appropriate compound (NO₂, NO or O₂) in the sample of the delivery gas (see, for example, Figure 11). The second sensor line (b) can act as confirm sensor line (see, for example, Figure 11). In other words, a detection signal from the second sensor line (b) can be compared to a detection signal from the first sensor line (a) to verify that the detection signal from the first sensor line (a) is accurate. An alternate embodiment of a platform including a sensor module is illustrated in Figure 11.

An additional advantage of having a second sensor line act as confirm sensor line is that the overall effect is similar to having continuous calibration. A detection signal from the confirm sensor that is similar to the detection signal from the alarm sensor line indicates that the sensors have not degraded, been damaged or broken. This configuration can also limit the number of times calibration needs to occur, as calibration may only be necessary when there is disagreement between the two sensors (e.g., detection signals differ more than a threshold value). Another advantage of having a second sensor line act as confirm sensor line is that it allows for increased precision in the amount of NO being delivered. An accurate NO reading allows the NO concentration to be honed in to the preset value(s) and stay on target (as discussed more above with the fine and gross tuning of NO delivery).

The comparison can be performed by a controller 613. In particular, the controller 613 can be configured to receive a first detection signal from at least one of the one or more sensors in a first sensor line (a) and a second detection signal from at least one of the one or more sensors in a second sensor line (b). The detection signal can depend on the sensor sending the signal. For example, a nitric oxide sensor can produce a nitric oxide detection signal, a nitrogen dioxide sensor can produce a nitrogen dioxide detection signal, and/or an oxygen sensor can produce an oxygen detection signal. The controller can compare the second detection signal with the first detection signal. If the second detection signal has a similar value to the first detection signal, then it can be assumed that both the first sensor line (a) and the second sensor line (b) are working properly. However, if the first detection signal and a second detection signal differ by greater than 2%, greater than 5%, greater than 10%, greater than 15% or greater than 20% of the first detection signal, then events, including activating an alert, closing or repositioning a valve or uncoupling a platform from the system, can occur.

The amount of nitric oxide, nitrogen dioxide and/or oxygen in a gas (i.e. first gas, second gas or delivery gas) gas can be predetermined. In some cases, the predetermined amount can be input into the controller 613. The controller 613 can compare the first detection signal and/or the second detection signal with a signal representing the predetermined amount. If the detection signal from the first sensor line (a) indicates that the amount of the appropriate compound (NO₂, NO or O₂) in the gas is differs from the predetermined amount by greater than 2%, greater than 5%, greater than 10%, greater than 15% or greater than 20% of the predetermined amount, several different events, including activating an alert, closing or repositioning a valve or uncoupling a platform from the system, can occur.

In more detail, an event can include an alert can be activated. An alert can include a sensory alarm. For example, an alert can include a sound. An alert can also include a visual element, such as flashing lights or text. An alert can include a somatosensory element, such as vibration.

Second, the position of at least one valve 614 within a platform 601, 602 can be changed. The position of the at least one valve 614 can be switched by a controller 613. The controller 613 can independently switch more than one valve 614. Each platform can include a valve 614 with at least two positions. The valve 614 can have an open position that allows a gas to pass from the platform 601, 602 through the valve 614 and into the delivery line 603. The valve 614 can also have a closed position that prevents a gas from passing from the platform 601, 602. If the detection signal from the first sensor line (a) indicates that the amount of the appropriate compound (NO₂, NO or O₂) in the sample of the delivery gas is different than the predetermined amount, at least one valve 614 in one of the platforms 601, 602 can be closed to prevent the gas in that platform 601, 602 from passing into the delivery line 603. In some cases, a valve 614 can be connected to a platform 601, 602 and a dump line 615. The valve 614 can have a first position that allows a gas to pass from the platform 601, 602 through the valve 614 and into a delivery line 603. The valve 614 can have a second position that allows a gas to pass from the platform 601, 602 through the valve 614 and into the dump line 615. A dump line 615 can discharge a gas in the platform 601, 602 out of the platform 601, 602 and prevent the gas from going into the delivery line 603.

Third, a platform 601, 602 can be uncoupled from the delivery line 603. In other words, the platform can be physically disconnected from the delivery line 603. This can prevent gas from be delivered to a patient. It can also allow the platform to be manipulated so that inspections or repairs of the platform can occur.

As shown in Figure 7, in some embodiments, a first sensor module 612a/b can be coupled to a first platform 601, and a second sensor module 612c/d can be coupled to a second platform 602. Like the sensor module 612 in Figure 6, the first sensor module 612a/b and/or the second sensor module 612c/d can each include a first sensor line (e.g. (a) or (c)) and a second sensor line (e.g. (b) or (d)). Each of the first sensor line (e.g. (a) or (c)) and the second sensor line (e.g. (b) or (d)) can include one or more nitric oxide sensors, one or more nitrogen dioxide sensors, or one or more oxygen sensors. A sample of the gas can be taken from the first platform 601 and directed into the first sensor module 612a/b. A first portion of the sample can be directed through the first sensor line (a). A second portion of the sample can be directed through the second sensor line (b). A sample of the gas can be taken from the second platform 602 and directed into the first sensor module 612c/d. A first portion of the sample can be directed through the first sensor line (c). A second portion of the sample can be directed through the second sensor line (d). As described above a first sensor line (e.g. (a) or (c)) can act as an alarm sensor line and a second sensor line (e.g. (b) or (d)) can act as a confirm sensor line.

Whether the sensor module is connected to a platform or the delivery line, a sensor module can include an exhaust 611 that expels the sample gas out of the sensor module. An exhaust can include a scrubber or absorbing material to eliminate any compounds in the expelled gas that may be dangerous or toxic. For example, an exhaust can include activated carbon, activated alumina or calcium sulfate.

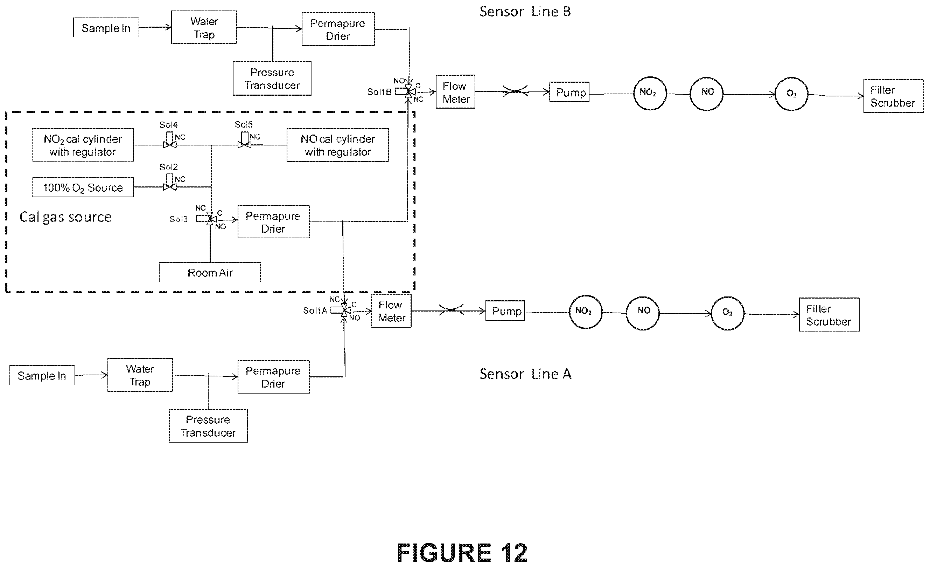

To ensure that a sensor module is operating correctly, the sensors in the sensor module can be calibrated. As shown in Figure 12, a dual system can be considered to include a first platform (platform A) and a second platform (platform B). A system can include two completely independent lines of sensors, one used for alarm (and control for NO) and the other set as a confirmation sensor. In Figure 12, only a single set of sensors for each system is shown. However, because each system can be interconnected by a USB connection, and the two independent controllers can be in communication with each other, an alternative logic can be to have one set from the dual platform system be assigned the alarm function, and the other set the confirm function.

To simplify the dual platform system, as shown in Figure 12 in the dotted box, there can only one set of calibration gases that can service both platforms. Not only can this configuration simplify the system, but having two independent calibration sources can decrease the reliability of the system. First, it can be desired to utilize the identical standard in calibrating all of the sensors in the dual system. Second, it can be easier to synchronize the calibrations in the dual system. Since all of the solenoids can be operated in the normally deactivated position for sampling, and because each system can have its unique three way solenoid to choose either sample or calibrate gases, i.e. SollA and Sol1B, each platform can be connected to the solenoids to activate them as necessary. With the proper filter, having one platform activate a solenoid may not result in damage in the other platform.

In order to insure the ability to monitor the sample gases during calibration, the master platform can schedule the calibrations. When the sensors on the slave platform are being calibrated, the confirm feature may not exist. When the master sensors are being calibrated, the slave's sensors can take up the alarm and control functions, and again, the confirm feature may not exist for a short time. Alternatively, each set of sensors can have a duplicate NO sensor, so that the control function would be unaffected during calibration. If there are two NO sensors on each platform, then the NO control function can be monitored by 4 sensors simultaneously, providing quadruple redundancy.

While the exemplary systems in the figures are shown with two identical platforms, it should be understood that the two platforms of a dual system can be different from one another. For example, a first platform can include a reservoir as a first source of nitric oxide, while a second platform can include a gas bottle as a second source of nitric oxide. As another example, a first platform can include a first source having nitric oxide-releasing compound, while a second platform can include a second source having nitric oxide. Variations in the system can depend on the needs of the particular user. For example, it can be quicker to provide nitric oxide from a gas bottle than to provide nitrogen dioxide from a reservoir. Therefore, it may be beneficial to include a gas bottle as part of a first platform that is acting as a backup to a second platform.

The platforms can also be substantially similar to one another. In other words, each platform can include the same significant features, but include slight variations in the components, such as tubing, valves, etc., that connect the significant features or the arrangement of the significant features.

### Examples

The dual (twin) platform device can include two completely independent NO inhalation devices, each which can stand by itself and can supply the complete patient's needs for NO inhalation gas. Because there can be two independent devices, the parallel platform can take over in the event of a failure. The FDA document for NO inhalation devices, dated January 24, 2000 has as an explicit requirement a completely independent backup system to provide NO inhalation gas in the event of failure of the primary device. Having a dual platform system can simplify the engineering of each independent subsystem, since each subsystem can qualify as the backup system for each other, and thus, an internal back up system for each platform may not be required.

The dual platform system can be designed to function as a single platform and can be virtually a doubly redundant platform. For a failure to occur, a component must fail in both independent systems at the same time. While theoretically possible, the odds of a two failures occurring at the same time in two independent systems can be extremely remote.

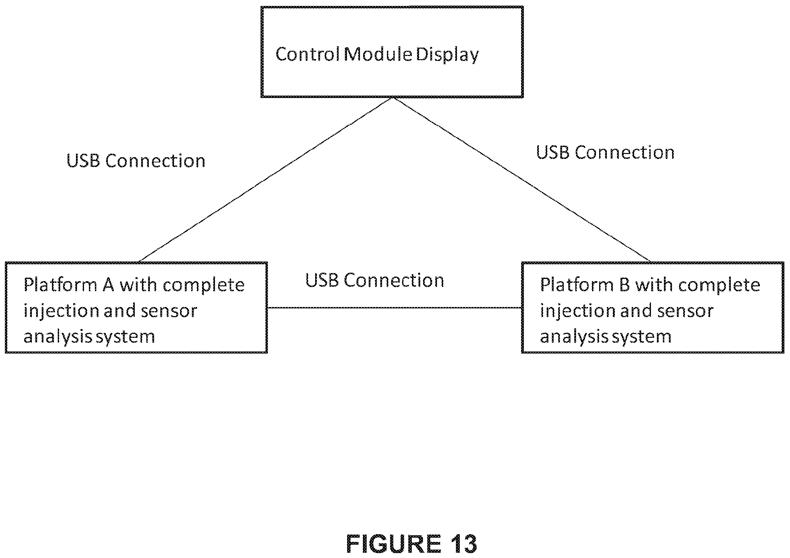

As shown in Figure 13, platform A and platform B can be identical in every respect. The interconnection between the two and to the common display module can be by means of USB cables or by a remote connection. Each system can include:
- Power supply with battery back-up.
- Twin sensors for parallel calibration (optional since the sensor system of one platform can act as the backup for the other). Each platform can also be equipped for automatic calibration. In order to allow for agreement between the two platforms, there may only be one set of calibration standards that both platforms share. If the calibration of the different sensors utilized different calibration standards, the calibration can be different for each sensor. If there were to be a failure, the system may need to be repaired. However, the calibration system may not shut down, and the system can continue to supply NO to the patient as required until the repair is completed. Repair can typically occur within a few hours, and thus, missing a calibration may not critical. In some cases, in order for a twin platform system to operate without conflicts, sensors in one sensor line can be characterized as the control/alarm (master) and the other line as the confirm (slave). Thus, except for the case when one system has failed, there may be no requirement to add dual parallel calibration sensors for each device.
- Source of nitric oxide, either from a bottle of compressed gas containing NO in nitrogen, NO₂ in air, nitrogen or oxygen, from a liquid source of N₂O₄, or from any other nitric oxide-releasing compound, as discussed further herein, that can produce dilute NO in a gas;
- An optional sample delivery connection to the gas line from the ventilator to the patient.
- A valve or other device for continuously withdrawing a gas sample for analysis, typically at a flow of about 120 ml/min. Each system can have its own independent sensors, liquid trap, sampling lines, and associated hardware and software for sensing the amount of nitrogen dioxide, nitric oxide and/or oxygen in the sample gas.
- A connection to a separate large display module for the health care personnel. Each platform can also have a small display in case the main large display fails. The small display can allow for complete monitoring and operation of the platform.
- The single large display can be connected to the twin platforms by cables; for example, USB cables (see Figure 13). This can allow the display to be placed remote from the platforms. It also allows the display to be placed at eye level, even on top of other equipment in the ICU or Operating theatre.

It can desirable and essential to operate both twin platforms in parallel and at the same time. Both can be producing some of the NO being delivered to the patient and both can be withdrawing a sample for analysis. In order to help with the diagnostic algorithms for determining which unit may be at fault in case of an overall system failure (too high or too low) the twin systems can be operated asymmetrically, namely one is providing say 80% of the needed NO and the other only 20% of the needed flow. This asymmetric balancing of the flows can have the following advantages as compared to having an equal contribution from both platforms:
- The platform that is contributing the larger fraction of the NO dose can be used for crude control. The platform proving the lower fraction of the total NO dose can be used for fine tuning the dose, thereby resulting in greater stability.
- In case of difficulty in determining which unit is at fault, a small change in one platform can be very different from a small change in the other.
- The platforms may not run empty at the same time.
Preferable splits can be 80: 20, or 1/3:2/3. Other split ratios may be used.

A key benefit can be that both platforms are up and running at all times. This can provide the following advantages:
- Each system can be fully calibrated and fully operational at all times.
- The operator can have the confidence that either system can take over the full load quickly.
- There may be no need to purge the patient delivery line or the gas sampling lines.
- No warm up time may be required.
- There may never be a point in time when no nitric oxide is being delivered to the patient, even in the event of a sudden catastrophic failure of one of the dual platforms.
- The overall dual system can provide true instantaneous back up at all times.
- If the backup mode is invoked automatically by the system, the backup mode can be started without operator intervention, and thus, can occur instantaneously.

In order to simplify the plumbing diagram figures to demonstrate the dual system, the calibration leg and the NO inhalation leg are drawn separately, but with indications where they connect as appropriate.

### A Dual Platform Liquid System

In a typical NO gas cylinder based platform, the concentration presented to the patient can be a function of the flow of the gas including nitric oxide into a ventilator output flow. The exact concentration can be determined by the dilution ratio of the supply gas to the total flow. For a liquid system (utilizing a reservoir), the control of the concentration presented to the patient can be virtually independent of the flow. The simplest control for a liquid source can be temperature, which can in turn control the pressure of the nitric oxide-releasing compound in the reservoir, and therefore, the concentration of nitric oxide-releasing compound released from the reservoir. The concentration of nitric oxide-releasing compound released can then affect the concentration of nitric oxide in a gas in a platform. For the liquid source, it can be unnecessary to control the flow of the gas through a platform, instead, only the temperature of the liquid source may need to be controlled. Furthermore, the combined flow from the twin platforms can be limited to less than 10% of the total flow presented to the patient so that the percentage oxygen may not be significantly diluted from that presented by the ventilator and also may not affect the pulsations from the ventilator. Thus, changing the flow in the platform can change the dilution ratio slightly, and the main control for the liquid system can be the temperature of the reservoir. A 10°C change in the temperature at which a reservoir operates can roughly double the concentration of nitric oxide released, giving a large dynamic range. As a result of the fact that the concentration can be a function of the reservoir temperature and not the injection flow, the precision required for the injection flow for a gas based system may not be required for the liquid based system. Thus, instead of an expensive mass flow controller, a pump with a pump controller can be utilized to vary the pumping speed and thus the gas flow. Also, the air flow can be controlled prior to the addition of the NO₂ gas, and thus, expensive materials that are compatible with NO₂ may not be required.

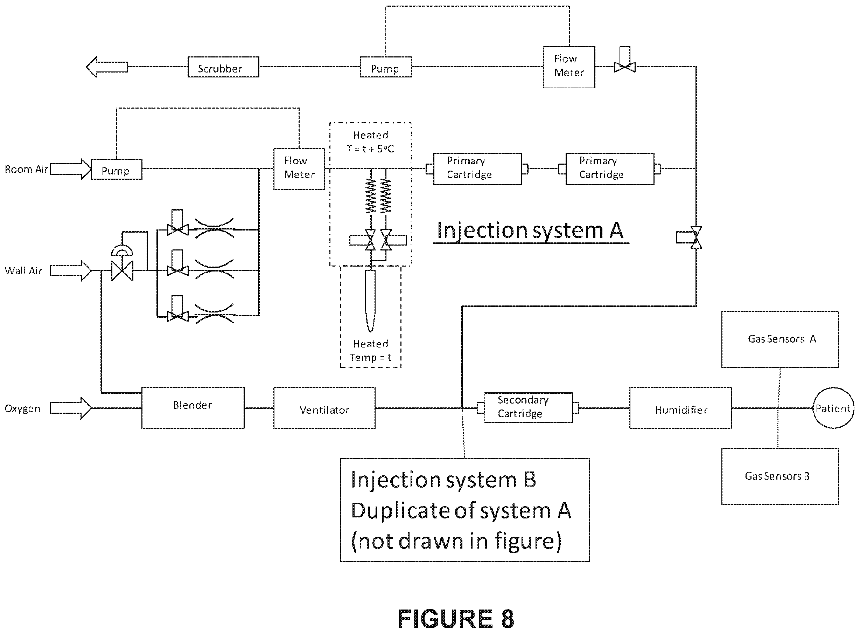

Figure 8 shows a detail of one of the platforms for a liquid system. The diagram of Figure 8 includes three options that may be included or not in a dual platform system. The first option can be a dump flow. The purpose of this option can be to allow for fast changes in concentration of a gas (e.g. NO₂ or NO) presented to the patient. The heat up and cool down rate of the reservoir can control the speed of changing the concentration. Instead of using a high capacity cooler to obtain a rapid decrease in concentration, the speed of decreasing the concentration of a liquid system can be made instantaneous with a dump valve. If it is desired to lower the concentration, the solenoid and pump in the dump system can be activated in order to dump or discharge whatever fraction of the gas may be required to achieve the desired lower concentration. Simultaneously, the temperature of the reservoir can start to lower. While the dump flow discharges gas to decrease in concentration and gets the concentration to a set point, the reservoir temperature can drop and can cause the NO₂ output from the reservoir to drop. Eventually, the dump flow can drop to zero (i.e. stop discharging gas) when the reservoir temperature reaches the desired temperature to support the set point. Thus, the dump flow can be a temporary component that can be used to quickly reduce the concentration until the reservoir temperature reaches the desired level.

A second option can be having two or more restrictors immediately after the reservoir. The exact output of the reservoir system can be set by the temperature of the reservoir and the flow characteristics of the restrictors. If the diameter of the restrictor is made smaller without changing the length, the flow can decrease. A larger the overall restriction can result in a larger the amount of NO₂ that passes through the restrictor at a given pressure. The pressure can be determined by the temperature of the reservoir. If the diameter is held constant and the length is increased, the flow can decrease; if the length is shortened, the flow can increase. By having two identical restrictors in parallel as shown in Figure 8, the system can increase the output concentration quickly (see also Figure 2). If the two restrictors are identical, then turning on the second one can double the output (it can be assumed for these discussions that the normal steady state condition is operating with the correct temperature and only one restrictor being used). If it is desired to increase the concentration to the patient quickly, using the scheme of Figure 8, the second restrictor can be activated, still keeping the first restrictor active. The dump flow can be activated to dump any excess material in order to reach the desired concentration. Immediately after activating the second restrictor, the system can begin to heat up the reservoir and using the dump flow to dump the excess material. Once the temperature of the reservoir is high enough to obtain the proper concentration with only one restrictor, the second restrictor can be closed, and the dump flow can be adjusted properly. Finally, when everything has stabilized at the new higher concentration, the second restrictor and dump flow can be turned off.

In order to achieve concentration increases greater than a factor of two, the second restrictor can have a large diameter or shorter length, so that the flow can increase by a different factor, for example, a factor of three, with the dump line and reservoir temperature control being utilized to achieve the desired concentration. Once the desired concentration has been reached, the second restrictor can be deactivated along with the dump line.

A third option included in the flow diagram of Figure 8 can be the connection to the wall air input at the facility along with more than one (e.g. 2 or 3) solenoid controlled restrictors. Three such solenoids controlled restrictors are shown. The solenoid controlled restrictors can be used to achieve the desired injection flow. The requirements can be that the injection flow needs to be 10% or less of the total flow to the patient from the combination of the twin systems. It may be desirable to operate the injection flow close to or almost at 10%. By having two solenoids and two restrictors can allow for three unique flows. Flow 1 can have only a single restrictor active, Flow 2 can have the other but different restrictor active, and Flow 3 can have both restrictors active. If more choices are required, three solenoid-restrictor combinations can be used.

It should be noted that the temperature of the N₂O₄ restrictor and controlling solenoids, those components within the dot-dashed box (upper), can be kept a few degrees higher than the controlling temperature of the N₂O₄ reservoir, components within the dashed box (lower), in order to keep N₂O₄ from condensing on the restrictor, solenoid or other associated plumbing within the box.

### Dual Platform System Flow System

Figure 8 shows a simplified diagram of a dual platform liquid system. It shows the detail of the injection module for platform A, but the sensor system of platform A is not shown in detail only by a single box indication. Figure 12 shows the details of the sensor module diagram, including sensor line A and sensor line B. The components of sensor line A of Figure 12 are shown as the contents of the box labeled "Gas Sensors A" in Figure 8. The components for the injection system of platform B can be identical to those of platform A, and thus, in Figure 8 are only shown as a single box.

As shown in Figure 8, the two platforms can include injection lines connecting directly to the delivery line, including a ventilator. Another configuration can be to have the two platforms teed together before the delivery line and only a single injection port used to connect the two platforms to the delivery line. Using a tee can reduce the plumbing lines but can add a catastrophic mode if the line were disconnected or crimped and the sensor on the system also failed. Having a separate delivery line for the delivery gas can adds quadruple redundancy. The same can be true for a sample line. Figure 8 shows each platform with a unique line to the sample port on the delivery line. Those two lines can be teed and only have one sample line at the delivery line. One disadvantage of that approach can be that if the sample line at the delivery line is disconnected, both sample platforms can be disconnected. If there were two unique lines, then it can be possible for one line to be disconnected but the second one still connected and usable. The same argument can be true for injection lines.

A solenoid can be located after a second primary cartridge, which can be a type of receptacle. A solenoid after the second primary cartridge but before a secondary cartridge (a type of receptacle) can be used to isolate the system from the delivery line. If a given platform goes off line due to a failure, this solenoid can close to isolate the platform from the rest of the system. The solenoid can also be used to allow for purging of the system upstream of the solenoid. If the system is operational and the solenoid is closed, but the dump line is active, it can be possible to flush a platform. This can be a desired activity, especially when the reservoir and/or primary cartridges need to be replaced.

The simplest implementation of the dual platform system can be for each platform to contain only a single reservoir system. Within the operating software of the dual platform system, there can be a command that could be activated when it is time to change one of the reservoirs. When activated, the system can increase the temperature of the reservoir in the first platform not being changed while decreasing the temperature of the reservoir in the second platform being changed, until such time as the first platform can supply the entire NO required. At that point a message could be issued to the user that the unit is ready for the reservoir to be changed. After the reservoir is changed, the operation can be reversed, i.e. the second platform would heat up and the first platform that was supplying the entire NO would cool down, until the condition was meet where the platforms were operating in the normal mode. If the reservoir in the first platform needed to be changed, the operation could repeat with the actions swapped for each platform. Since a standard amount of N₂O₄ can be added to each reservoir, and because the controller can keep a history file of the temperature of the reservoir as a function of time, the controller can estimate the amount of N₂O₄ used, and can issue a message significantly before a reservoir ran dry that the reservoir needed to be replaced.

The nature of the reservoir filled with N₂O₄ can be completely different than the gas in an automobile. The car has a basically binary system; a car can run until it runs out of gas, and then the car can stop. For a reservoir, because the N₂O₄ can be under pressure, the output can gradually drop. This drop can be detected by the sensors, especially if a high level NO sensor is used just before the injection port. Under this condition, the second platform can increase its output, while an error message can be issued to the operator to replace the emptying vessel.

A high concentration NO sensor can be used after a primary cartridge. That sensor can help achieve even faster response times because, without the sensor, any change may not be detected until a gas flows through and completely flushes the secondary cartridge. If on the other hand, the exact concentration of NO from a platform into a delivery line is monitored, a more precise and faster control of the dump system can be achieved. Since it is desired not to operate these electrochemical sensors with "bone dry" gases, a Permapure Nafion drier can be added just before the sensor to equilibrate the gas to the atmospheric humidity.

It should be emphasized that if one unit fails and the operator needs to go into the "backup" mode that is a temporary operation, a spare platform can be used to replace the failed platform. This can be true if the individual platform modules are not designed to support two reservoir systems.

### Full Automatic Calibration for Platforms

The calibration gas used for the low level calibration can typically be room air, which can include less than 0.1 PPM NO or NO₂ and can include about 21% oxygen. The high level calibration can typically be performed at about 10 PPM NO₂, 45 PPM NO and 100% oxygen, with standard gases supplied from certified cylinders containing the desired gas and concentration. Oxygen can be supplied by the hospital. The high level calibration can normally be performed monthly, or whenever a new patient may be connected to the device, whichever comes first. More frequent calibration can be essential because, without calibration, a user may not know if a sensor has failed. Automatic calibration can be essential in a double redundant system so as to avoid the failure of a sensor causing a shut-down, which can be fatal. In principle, manual calibration can also suffice, but automatic calibration can be carried out often and automatically.

The scheme shown in Figure 11 can address the known inadequacies of the current commercial systems in use to supply NO inhalation gas to patients that are on a ventilator to assist with breathing. Specifically, current commercial system can have four shortcomings that can be solved by automatic calibration:
- The ability to monitor the sample during calibration and not being off line while injecting NO gas.
- The ability to perform both low and high level calibration automatically.
- Dual sensors to allow for complete redundancy in the event one sensor fails.
- The ability to inject a pulse of calibration gas into the sample line as a means of checking on the validity of the calibration. The correct peak height and the height of the transient peak are used as a quick check that can be carried out frequently, even hourly, if needed.

### Monitoring during calibration

The current systems may need to go off line in order to perform a daily low level calibration. The current system is disclosed in US Patent 5,752,504, a patent of one of the suppliers of such a device including a calibration system. During calibration with their protocol, the set point cannot be changed nor is the sample being monitored for NO or NO₂ while calibration is occurring. If a failure were to occur during the calibration cycle, the patient may be unprotected. Another drawback can be that because of the risk of failure during calibration, the calibrations can be carried out only once per month. Followed the scheme of Figure 11, a calibration system can calibrate different sensors while simultaneously monitoring for a variety of gases. As described below, a sensor module can include at least two sensors for each gas (NO₂, NO and/or O₂), one sensor to monitor the gas for alarm purposes (and control, in the case of NO) and one sensor to confirm the alarm sensor reading. By having two sensors for each gas, a sensor module can be redundant, which significantly increases reliability. Moreover, during the five to ten minutes required to calibrate the sensors, the current systems may be unable to detect a catastrophic failure or unacceptable system drift. Using a sensor module and calibration standards, as shown in Figure 11, these issues can be avoided.

Table 1 summarizes the different solenoid activations to perform the different tasks.

**Table 1 - Solenoid Position for Different Functions**

| | **Solenoid Position for Different Functions (0 = deactivated)** | | | | | | | | |
|---|---|---|---|---|---|---|---|---|---|
| | **Sample** | **Low Level Cal** - **Alarm** | **NO High Level Cal - Alarm** | **NO₂ High Level Cal - Alarm** | **O₂ High Level Cal - Alarm** | **Low Level Cal - Confirm** | **NO High Level Cal - Confirm** | **NO₂ High Level Cal - Confirm** | **O₂ High Level Cal**- **Confirm** |
| **Sol 1** | 0 | 1 | 1 | 1 | 1 | 0 | 0 | 0 | 0 |
| **Sol 2** | 0 | 0 | 0 | 0 | 0 | 1 | 1 | 1 | 1 |
| **Sol 3** | 0 | 1 | 0 | 0 | 0 | 1 | 0 | 0 | 0 |
| **Sol 4** | 0 | 0 | 0 | 1 | 0 | 0 | 0 | 1 | 0 |
| **Sol 5** | 0 | 0 | 1 | 0 | 0 | 0 | 1 | 0 | 0 |
| **Sol 6** | 0 | 0 | 0 | 0 | 1 | 0 | 0 | 0 | 1 |

As seen in Figure 11, there can be two completely independent sensor lines, one for the alarm sensors and one for the confirm sensors. During calibration of the alarm sensors, the confirm sensors can take over the alarm functionality, and there may not be a confirm functionality. During calibration of the confirm sensors, there may not be a confirm functionality. Thus, there may not be any point in time where a gas being presented to a patient is not being monitored. An additional advantage of the ability to monitor during calibration can be the ability to perform auto-calibration. This can be advantageous because it may not be advisable to perform an auto-calibration with the monitoring sensors being off-line and/or not having a health professional monitoring the patient during the calibration procedure.

For an NO sensor, another operational mode that can be used is for one sensor to monitor and control, while the back-up sensor can be used as a check on the accuracy of the other sensor. If a fault is detected, the remaining "good" sensor can take over both functions. Since the NO level can be calculated from the NO concentration and the known constant flow through a mass flow controller, deviation from the calculated value can be used to help identify the sensor that has failed.

### Performing Auto-calibration

As noted above, a low level calibration can be performed daily. A high level calibration can be performed whenever the confirm sensor reading differed from the alarm sensor reading by the amount setup in the configuration file of the system. Since the FDA requires the reading to be within +/- 20% of actual, a preferred setup in a configuration file can be +/- 10% to allow for a significant safety factor. These devices can be used on humans who are quite ill, and can require constant attention by the medical staff. The medical staff in a typical intensive care unit, where these devices are being used, can have many tasks to perform. Any manual task that the staff is currently performing that can be made automatic can reduce the work load on the medical staff, as well as decrease the potential for error by this overworked staff.

As previously mentioned, Table 1 summarizes the different solenoid states of the solenoids in Figure 11 that can be used to perform sampling and the different calibrations. As noted in Table 1, a low level gas can be room air. Calibration can be commanded in a variety of ways. It can be commanded manually, with the operator activating the appropriate calibrate button, either low or high calibration. Alternatively, it can be performed on a scheduled timed manner. For example, the low level calibration can occur at 10:00 am every day, and the high level calibration can occur at a lower frequency.

In all cases of calibration, i.e. low and high level, the system can be programmed to allow sufficient time once the different solenoids are open to completely flush the system, and therefore, only present the different sensors with the proper calibration sample.

An alternative approach for auto calibration can be for the controller to send a signal to a specific solenoid to open the valves momentarily so as to obtain a short burst of the calibration gas. With an accurately timed sequence and a known rise time of the sensors, the momentary peak height can be used to check that the sensors are properly calibrated. The controller can command the momentary injection of calibration gas, and the time from injection to detection can also be used for calibration purposes. A momentary pulsed calibration can use only a tiny amount of gas. One advantage of the momentary pulse of calibration gas can be the preservation of gas, which can allow for much more frequent auto calibrations. Instead of once a month as is the current requirement, calibration can be performed daily, hourly, or even continuously, if needed. A second advantage can be that the momentary pulse can sit on top of the background signal, and therefore, the two signals can be readily separated. The controller can fire the pulse and can predict exactly when to expect the peak and the size of the peak. A longer pulse can give rise to a larger peak height. This momentary calibration can be used in place of constant steady state calibration, which can be wasteful of the calibration gas.

### Dual sensor for redundancy

As seen from Figure 11, there can be two completely independent sensor lines (i.e. manifolds) for monitoring the NO, NO₂ and O₂ levels. If there is a failure of any component in either of these sensor lines, an error message would be issued to the operator, but the unit can be able to continue operating without any adverse effect on the patient. This is not the case for the current system in the field. Thus, for example, if there were a pump failure on either line, the controller can receive a signal from the appropriate pump control module (called flow meter in diagram), and can issue an error message to the user. If the error occurred in the confirm line, the confirm function can be lost, but as described above that may not be a critical failure and the unit can continue operating properly. If the error occurred in the alarm line, the confirm line can take over the alarm function for NO, NO₂ and O₂, as well as the control function for NO, and again the confirm function may not be available.

If there is a complete failure of one of the sensors, which can be evidenced by a zero or full scale signal, for example, the sensor giving the error can be taken off line and that function can be taken over by the other sensor.

The other components in Figure 11 that are not completely duplicated are passive components. Passive components can include a water trap, a pressure transducer, a Permapure drier and a filter scrubber at the output. Optionally, a system can include two Permapure driers placed immediately after Sol1 and Sol2. It may not be desirable to include two pressure transducers because that can cut the signal in half from each transducer and can increase the signal-to-noise ratio. While a pressure transducer can be included, it may not be a critical component. The unit can operate fully without any adverse effect on the patient without it, because the information obtained from the pressure transducer can be inferred from the other sensors, but with a time delay of a minute or two. If there is only a single pressure transducer, that can imply inclusion of a single water trap. Because this is a highly reliable passive component and because it would be more efficient in removing water at the higher flow than the lower flow that can occur with two traps, there may not be advantage to having a redundant system for a water trap or for a pressure transducer.

Details of one or more embodiments are set forth in the accompanying drawings and description. Other features, objects, and advantages will be apparent from the description, drawings, and claims. Although a number of embodiments of the invention have been described, it will be understood that various modifications may be made without departing from scope of the invention. It should also be understood that the appended drawings are not necessarily to scale, presenting a somewhat simplified representation of various features and basic principles of the invention.

## Claims

1. A system for the delivery of nitric oxide, comprising:
a first platform (601) including a first source of nitric oxide (606), wherein the first source of nitric oxide (606) includes nitric oxide, wherein the first platform (601) comprises a first heating device (204) and the first heating device (204) is thermally connected with a reservoir (200) of the first source of nitric oxide (606), a second platform (602) including a second source of a nitric oxide (606), wherein the second platform (602) comprises a second heating device (204) and the second heating device (204) is thermally connected with a reservoir (200) of the second source of nitric oxide (606),
a delivery line (603) coupled to the first platform (601) and the second platform (602), and
a controller (206) configured to operate the first platform (601) and the second platform (602) simultaneously by controlling a temperature of the first source (606) and the second source (606), and
a sensor module (612) including one or more nitric oxide sensors and one or more nitrogen dioxide sensors, wherein the sensor module includes a first sensor line including one or more nitric oxide sensors (609) and a second sensor line including one or more nitric oxide sensors (609) and one or more oxygen sensors (610).

2. The system of claim 1, wherein the system includes one or more receptacles (605)including a support and a reducing agent capable of converting a nitric oxide-releasing compound to nitric oxide.

3. The system of claim 1 or claim 2, wherein the first source of nitric oxide (606) includes a first nitric oxide-releasing compound, preferably wherein the first nitric oxide-releasing compound includes nitrogen dioxide.

4. The system of any one of claims 1-3, wherein the second source of nitric oxide (606) includes a second nitric oxide-releasing compound, preferably wherein the first nitric oxide-releasing compound and the second nitric oxide-releasing compound are the same compound or wherein the second nitric oxide-releasing compound includes nitrogen dioxide or wherein the second source of nitric oxide (606) includes nitric oxide.

5. The system of any one of claims 1-4, wherein each reservoir (200) includes dinitrogen tetroxide and/or wherein each reservoir (200) includes one or more restrictors (201), preferably two or more restrictors (201).

6. The system of claim 5, wherein each restrictor (201) has a length, and the length of a first restrictor (201) is substantially identical to the length of a second restrictor (201) or wherein the length of a first restrictor (201) is different than the length of a second restrictor (201).

7. The system of claim 5 or claim 6, wherein each restrictor (201) has an internal diameter, and the internal diameter of a first restrictor (201) is substantially identical to the internal diameter of a second restrictor (201) or the internal diameter of a first restrictor (201) is different than the internal diameter of a second restrictor (201).

8. The system of any one of claims 4-7, wherein each restrictor (201) includes a valve (614) having an open position that allows a gas to pass through the restrictor (201) and a closed position that prevents a gas from passing through the restrictor (201), preferably wherein the controller (206) is configured to independently switch the valve on each restrictor (201) between the open position and the closed position.

9. The system of any one of claims 1-8, wherein the controller (206) is configured to receive a temperature input representing a first predetermined temperature at which the first reservoir (200) should operate and/or wherein the controller (206) is configured to receive a temperature input representing a second predetermined temperature at which the second reservoir (200) should operate.

10. The system of any one of claims 1-9, wherein the first heating device (204) is thermally connected with one or more restrictors (201) of the reservoir (200) of the first source of nitric oxide and/or wherein the second heating device (204) is thermally connected with one or more restrictors (201) of the reservoir (200) of the second source of nitric oxide (606).

11. The system of any one of claims 3-10, wherein the first platform (601) comprises a first receptacle (605) coupled to the first source of nitric oxide (606), wherein the first receptacle(605) includes a first support and a first reducing agent capable of converting the first nitric oxide-releasing compound to nitric oxide and/or wherein the second platform comprises a second receptacle coupled to the second source of nitric oxide, wherein the second receptacle (605) includes a second support and a second reducing agent capable of converting the second nitric oxide-releasing compound to nitric oxide.

12. The system of any one of claims 1-11, wherein the sensor module (612) is coupled to the delivery line and/or wherein the controller (206) is configured to receive a detection signal from at least one of the one or more nitric oxide sensors.

13. The system of claim 12, wherein wherein the controller (206) is configured to receive a first detection signal from at least one of the one or more nitric oxide sensors in the first sensor line and a second detection signal from at least one of the one or more nitric oxide sensors in the second sensor line.

14. The system of claim 13, wherein the controller (206) is configured to receive a nitric oxide-signal representing a predetermined amount of nitric oxide to be delivered via the delivery line, preferably wherein the controller (206) is configured to compare each of the first detection signal and the second detection signal to the nitric oxide-signal, and to activate an alert if the first detection signal or the second detection signal differ from the nitric oxide-signal by greater than 5% of the nitric oxide-signal or if the first detection signal or the second detection signal differ from the nitric oxide-signal by greater than 10% of the nitric oxide-signal.

15. The system of any one of claims 1-14, wherein the first platform (601) comprises a first flow valve prior to the delivery line, the first flow valve having a first position configured to allow the first gas to pass from the first platform into the delivery line, and a second position configured to allow the first gas to pass out of the first platform via a first dump line or wherein the system comprises a first flow valve in the first platform prior to the delivery line, the first flow valve having an open position configured to allow the first gas to pass from the first platform into the delivery line and a closed position configured to prevent the first gas from passing from the first platform into the delivery line.

16. The system of any one of claims 1-15, wherein the system comprises a first dump line (615) coupled to the first platform by a first dump valve, the first dump valve having an open position configured to allow the first gas to pass from the first platform into the first dump line (615) and a closed position configured to prevent the first gas from passing into the first dump line (615).

17. The system of any one of claims 1-16, wherein the second platform (602) comprises a second flow valve prior to delivery line that has a first position configured to allow the second gas to pass from the second platform into the delivery line, and a second position configured to allow the second gas to pass out of the second platform (602) via a dump line (615).

18. The system of any one of claims 1-17, further comprising a set of nitric oxide calibration standards including at least one nitric oxide calibration fluids, each nitric oxide calibration fluid having a different known amount of nitric oxide, preferably wherein the controller is configured to switch the first flow valve from the open position to the closed position if at least one of the one or more nitric oxide sensors in the first platform (601) detects an amount of nitric oxide gas in one of the at least two nitric oxide calibration fluids that differs by 10% or more from the known amount of nitric oxide in the at least one nitric oxide calibration fluid and/or, wherein the controller is configured to switch the second valve from the open position to the closed position if at least one of the one or more nitrogen dioxide sensors in the second platform (602) detects an amount of nitric oxide gas in one of the at least two nitric oxide calibration fluids that differs by 10% or more from the known amount of nitric oxide in the at least one nitric oxide calibration fluid and further comprising a set of nitrogen dioxide calibration standards including at least one nitrogen dioxide calibration fluids, each nitrogen dioxide calibration fluid having a different known amount of nitrogen dioxide and/or a set of oxygen calibration standards including at least one oxygen calibration fluids, each oxygen calibration fluid having a different known amount of oxygen.

## Patentansprüche

1. System zur Abgabe von Stickoxid, umfassend:
eine erste Plattform (601), die eine erste Quelle für Stickoxid (606) einschließt, wobei die erste Quelle für Stickoxid (606) Stickoxid einschließt, wobei die erste Plattform (601) eine erste Heizvorrichtung (204) umfasst, und die erste Heizvorrichtung (204) thermisch mit einem Reservoir (200) der ersten Quelle für Stickoxid (606) verbunden ist,
eine zweite Plattform (602), die eine zweite Quelle für ein Stickoxid (606) einschließt, wobei die zweite Plattform (602) eine zweite Heizvorrichtung (204) umfasst, und die zweite Heizvorrichtung (204) thermisch mit einem Reservoir (200) der zweiten Quelle für Stickoxid (606) verbunden ist,
eine Abgabeleitung (603), die mit der ersten Plattform (601) und der zweiten Plattform (602) gekoppelt ist, und
eine Steuerung (206), die ausgelegt ist, um die erste Plattform (601) und die zweite Plattform (602) simultan zu betreiben, indem eine Temperatur der ersten Quelle (606) und der zweiten Quelle (606) gesteuert wird, und
ein Sensormodul (612), das ein oder mehrere Stickoxidsensoren und einen oder mehrere Stickstoffdioxidsensoren einschließt, wobei das Sensormodul eine erste Sensorleitung, die einen oder mehrere Stickoxidsensoren (609) einschließt, und eine zweite Sensorleitung einschließt, die einen oder mehrere Stickoxidsensoren (609) und einen oder mehrere Sauerstoffsensoren (610) einschließt.

2. System nach Anspruch 1, wobei das System einen oder mehrere Behälter (605) einschließt, die einen Träger und ein Reduktionsmittel einschließen, welches in der Lage ist, eine Stickoxid freisetzende Verbindung in Stickoxid umzuwandeln.

3. System nach Anspruch 1 oder Anspruch 2, wobei die erste Quelle für Stickoxid (606) eine erste Stickoxid freisetzende Verbindung einschließt, wobei die erste Stickoxid freisetzende Verbindung vorzugsweise Stickstoffdioxid einschließt.

4. System nach einem der Ansprüche 1 bis 3, wobei die zweite Quelle für Stickoxid (606) eine zweite Stickoxid freisetzende Verbindung einschließt, wobei die erste Stickoxid freisetzende Verbindung und die zweite Stickoxid freisetzende Verbindung vorzugsweise dieselbe Verbindung sind, oder wobei die zweite Stickoxid freisetzende Verbindung Stickstoffdioxid einschließt, oder wobei die zweite Quelle für Stickoxid (606) Stickoxid einschließt.

5. System nach einem der Ansprüche 1 bis 4, wobei jedes Reservoir (200) Distickstofftetroxid einschließt, und/oder wobei jedes Reservoir (200) ein oder mehrere Durchflussbegrenzer (201) einschließt, vorzugsweise zwei oder mehr Durchflussbegrenzer (201).

6. System nach Anspruch 5, wobei jeder Durchflussbegrenzer (201) eine Länge aufweist, und die Länge eines ersten Durchflussbegrenzers (201) mit der Länge eines zweiten Durchflussbegrenzers (201) im Wesentlichen identisch ist, oder wobei die Länge eines ersten Durchflussbegrenzers (201) von der Länge eines zweiten Durchflussbegrenzers (201) verschieden ist.

7. System nach Anspruch 5 oder Anspruch 6, wobei jeder Durchflussbegrenzer (201) einen Innendurchmesser aufweist, und der Innendurchmesser eines ersten Durchflussbegrenzers (201) mit dem Innendurchmesser eines zweiten Durchflussbegrenzers (201) im Wesentlichen identisch ist, oder wobei der Innendurchmessers eines ersten Durchflussbegrenzers (201) von dem Innendurchmesser eines zweiten Durchflussbegrenzers (201) verschieden ist.

8. System nach einem der Ansprüche 4 bis 7, wobei jeder Durchflussbegrenzer (201) ein Ventil (614) einschließt, das eine offene Position, in der Gas durch den Durchflussbegrenzer (201) hindurch gelangen kann, und eine geschlossene Position aufweist, die verhindert, dass ein Gas durch den Durchflussbegrenzer (201) gelangen kann, wobei die Steuerung (206) vorzugsweise ausgelegt ist, um das Ventil an jedem Durchflussbegrenzer (201) unabhängig zwischen der offenen Position und der geschlossenen Position zu schalten.

9. System nach einem der Ansprüche 1 bis 8, wobei die Steuerung (206) ausgelegt ist, um eine Temperatureingabe zu empfangen, die eine erste vorbestimmte Temperatur repräsentiert, bei der das erste Reservoir (200) betrieben werden sollte, und/oder wobei die Steuerung (206) ausgelegt ist, um eine Temperatureingabe zu empfangen, die eine zweite vorbestimmte Temperatur repräsentiert, mit der das zweite Reservoir (200) betrieben werden sollte.

10. System nach einem der Ansprüche 1 bis 9, wobei die erste Heizvorrichtung (204) thermisch mit einem oder mehreren Durchflussbegrenzern (201) des Reservoirs (200) der ersten Quelle für Stickoxid verbunden ist, und/oder wobei die zweite Heizvorrichtung (204) thermisch mit einem oder mehreren Durchflussbegrenzern (201) des Reservoirs (200) der zweiten Quelle für Stickoxid (606) verbunden ist.

11. System nach einem der Ansprüche 3 bis 10, wobei die erste Plattform (601) einen ersten Behälter (605) umfasst, der an die erste Quelle für Stickoxid (606) gekoppelt ist, wobei der erste Behälter (605) einen ersten Träger und ein erstes Reduktionsmittel einschließt, das in der Lage ist, die erste Stickoxid freisetzende Verbindung in Stickoxid umzuwandeln, und/oder wobei die zweite Plattform einen zweiten Behälter umfasst, der an die zweite Quelle für Stickoxid gekoppelt ist, wobei der zweite Behälter (605) einen zweiten Träger und ein zweites Reduktionsmittel einschließt, das in der Lage ist, die zweite Stickoxid freisetzende Verbindung in Stickoxid umzuwandeln.

12. System nach einem der Ansprüche 1 bis 11, wobei das Sensormodul (612) an die Abgabeleitung gekoppelt ist, und/oder wobei die Steuerung (206) ausgelegt ist, um ein Detektierungssignal von mindestens einem des einen oder der mehreren Stickoxidsensoren zu empfangen.

13. System nach Anspruch 12, wobei die Steuerung (206) ausgelegt ist, um ein erstes Detektierungssignal von mindestens einem von dem einen oder den mehreren Stickoxidsensoren in der ersten Sensorleitung und ein zweites Detektierungssignal von mindestens einem von dem einen oder den mehreren Stickoxidsensoren in der zweiten Sensorleitung zu empfangen.

14. System nach Anspruch 13, wobei die Steuerung (206) ausgelegt ist, um ein Stickoxidsignal zu empfangen, das eine vorbestimmte Menge an Stickoxid repräsentiert, die über die Abgabeleitung abgegeben werden soll, wobei die Steuerung (206) vorzugsweise ausgelegt ist, um jedes von dem ersten Detektierungssignal und dem zweiten Detektierungssignal mit dem Stickoxidsignal zu vergleichen, und einen Alarm zu aktivieren, wenn sich das erste Detektierungssignal oder das zweite Detektierungssignal von dem Stickoxidsignal um mehr als 5 % des Stickoxidsignals unterscheidet, oder wenn sich das erste Detektierungssignal oder das zweite Detektierungssignal von dem Stickoxidsignal um mehr als 10 % des Stickoxidsignals unterscheidet.

15. System nach einem der Ansprüche 1 bis 14, wobei die erste Plattform (601) ein erstes Durchflussventil vor der Abgabeleitung umfasst, das erste Durchflussventil eine erste Position, die ausgelegt ist, so dass das erste Gas von der ersten Plattform in die Abgabeleitung gelangen kann, und eine zweite Position aufweist, die ausgelegt ist, so dass das erste Gas über eine erste Abfallleitung aus der ersten Plattform heraus gelangen kann, oder wobei das System ein erstes Durchflussventil in der ersten Plattform vor der Abgabeleitung umfasst, wobei das erste Durchflussventil eine offene Position, die ausgelegt ist, so dass das erste Gas von der ersten Plattform in die Abgabeleitung gelangen kann, und eine geschlossene Position aufweist, die ausgelegt ist, um zu verhindern, dass das erste Gas von der ersten Plattform in die Abgabeleitung gelangt.

16. System nach einem der Ansprüche 1 bis 15, wobei das System eine erste Abfallleitung (615) umfasst, die über ein erstes Abfallventil an die erste Plattform gekoppelt ist, wobei das erste Abfallventil eine offene Position, die ausgelegt ist, so dass das erste Gas von der ersten Plattform in die erste Abfallleitung (615) gelangen kann, und eine geschlossene Position aufweist, die ausgelegt ist, um zu verhindern, dass das erste Gas in die erste Abfallleitung (615) gelangt.

17. System nach einem der Ansprüche 1 bis 16, wobei die zweite Plattform (602) ein zweites Durchflussventil vor der Abgabeleitung umfasst, das eine erste Position, die ausgelegt ist, so dass das zweite Gas aus der zweiten Plattform in die Abgabeleitung gelangen kann, und eine zweite Position aufweist, die ausgelegt ist, so dass das zweite Gas über eine Abfallleitung (615) aus der zweiten Plattform (602) heraus gelangen kann.

18. System nach einem der Ansprüche 1 bis 17, ferner umfassend einen Satz von Stickoxidkalibrierungsstandards, die mindestens ein Stickoxidkalibrierungsfluide einschließen, wobei jedes Stickoxidkalibrierungsfluid eine andere bekannte Menge an Stickoxid aufweist, wobei die Steuerung vorzugsweise ausgelegt ist, um das erste Durchflussventil von der offenen Position in die geschlossene Position zu schalten, wenn mindestens einer von dem einen oder den mehreren Stickoxidsensoren in der ersten Plattform (601) eine Menge an Stickoxidgas in einem von den mindestens zwei Stickoxidkalibrierungsfluiden detektiert, die sich um 10 % oder mehr von der bekannten Menge an Stickoxid in dem mindestens einen Stickoxidkalibrierungsfluid unterscheidet, und/oder wobei die Steuerung ausgelegt ist, um das zweite Ventil von der offenen Position in die geschlossene Position zu schalten, wenn mindestens einer von dem einen oder den mehreren Stickstoffdioxidsensoren in der zweiten Plattform (602) eine Menge an Stickoxidgas in einem von den mindestens zwei Stickoxidkalibrierungsfluiden detektiert, die sich um 10 % oder mehr von der bekannten Menge an Stickoxid in dem mindestens einen Stickoxidkalibrierungsfluid unterscheidet, und ferner umfassend einen Satz von Stickstoffdioxidkalibrierungsstandards, die mindestens ein Stickstoffdioxidkalibrierungsfluid einschließen, wobei jedes Stickstoffdioxidkalibrierungsfluid eine andere bekannte Menge an Stickstoffdioxid aufweist, und/oder einen Satz von Sauerstoffkalibrierungsstandards, die mindestens ein Sauerstoffkalibrierungsfluid einschließen, wobei jedes Sauerstoffkalibrierungsfluid eine andere bekannte Sauerstoffmenge aufweist.

## Revendications

1. Système de distribution d'oxyde nitrique, comprenant :
une première plate-forme (601) comportant une première source d'oxyde nitrique (606), la première source d'oxyde nitrique (606) comportant de l'oxyde nitrique, la première plate-forme (601) comprenant un premier dispositif de chauffage (204) et le premier dispositif de chauffage (204) étant relié thermiquement à un réservoir (200) de la première source d'oxyde nitrique (606), une deuxième plate-forme (602) comportant une deuxième source d'oxyde nitrique (606), la deuxième plate-forme (602) comprenant un deuxième dispositif de chauffage (204) et le deuxième dispositif de chauffage (204) étant relié thermiquement à un réservoir (200) de la deuxième source d'oxyde nitrique (606),
une ligne de distribution (603) couplée à la première plate-forme (601) et la deuxième plate-forme (602), et
un contrôleur (206) configuré pour faire fonctionner simultanément la première plate-forme (601) et la deuxième plate-forme (602) en régulant une température de la première source (606) et de la deuxième source (606), et
un module de capteurs (612) comportant un ou plusieurs capteurs d'oxyde nitrique et un ou plusieurs capteurs de dioxyde d'azote, le module de capteurs comportant une première ligne de capteurs comportant un ou plusieurs capteurs d'oxyde nitrique (609) et une deuxième ligne de capteurs comportant un ou plusieurs capteurs d'oxyde nitrique (609) et un ou plusieurs capteurs d'oxygène (610).

2. Système de la revendication 1, le système comportant un ou plusieurs récipients (605) comportant un support et un réducteur pouvant transformer un composé libérant de l'oxyde nitrique en oxyde nitrique.

3. Système de la revendication 1 ou la revendication 2, dans lequel la première source d'oxyde nitrique (606) comporte un premier composé libérant de l'oxyde nitrique, de préférence dans lequel le premier composé libérant de l'oxyde nitrique comporte du dioxyde d'azote.

4. Système de l'une quelconque des revendications 1 à 3, dans lequel la deuxième source d'oxyde nitrique (606) comporte un deuxième composé libérant de l'oxyde nitrique, de préférence dans lequel le premier composé libérant de l'oxyde nitrique et le deuxième composé libérant de l'oxyde nitrique sont le même composé ou dans lequel le deuxième composé libérant de l'oxyde nitrique comporte du dioxyde d'azote ou dans lequel la deuxième source d'oxyde nitrique (606) comporte de l'oxyde nitrique.

5. Système de l'une quelconque des revendications 1 à 4, dans lequel chaque réservoir (200) comporte du tétroxyde de diazote et/ou dans lequel chaque réservoir (200) comporte un ou plusieurs limiteurs de débit (201), de préférence au moins deux limiteurs de débit (201) .

6. Système de la revendication 5, dans lequel chaque limiteur de débit (201) a une longueur, et la longueur d'un premier limiteur de débit (201) est sensiblement identique à la longueur d'un deuxième limiteur de débit (201) ou dans lequel la longueur d'un premier limiteur de débit (201) est différente de la longueur d'un deuxième limiteur de débit (201).

7. Système de la revendication 5 ou la revendication 6, dans lequel chaque limiteur de débit (201) a un diamètre interne, et le diamètre interne d'un premier limiteur de débit (201) est sensiblement identique au diamètre interne d'un deuxième limiteur de débit (201) ou le diamètre interne d'un premier limiteur de débit (201) est différent du diamètre interne d'un deuxième limiteur de débit (201).

8. Système de l'une quelconque des revendications 4 à 7, dans lequel chaque limiteur de débit (201) comporte une vanne (614) ayant une position ouverte qui permet à un gaz de traverser le limiteur de débit (201) et une position fermée qui empêche un gaz de traverser le limiteur de débit (201), de préférence dans lequel le contrôleur (206) est configuré pour basculer indépendamment la vanne de chaque limiteur de débit (201) entre la position ouverte et la position fermée.

9. Système de l'une quelconque des revendications 1 à 8, dans lequel le contrôleur (206) est configuré pour recevoir une entrée de température représentant une première température prédéterminée à laquelle le premier réservoir (200) devrait fonctionner et/ou dans lequel le contrôleur (206) est configuré pour recevoir une entrée de température représentant une deuxième température prédéterminée à laquelle le deuxième réservoir (200) devrait fonctionner.

10. Système de l'une quelconque des revendications 1 à 9, dans lequel le premier dispositif de chauffage (204) est relié thermiquement à un ou plusieurs limiteurs de débit (201) du réservoir (200) de la première source d'oxyde nitrique et/ou dans lequel le deuxième dispositif de chauffage (204) est relié thermiquement à un ou plusieurs limiteurs de débit (201) du réservoir (200) de la deuxième source d'oxyde nitrique (606).

11. Système de l'une quelconque des revendications 3 à 10, dans lequel la première plate-forme (601) comprend un premier récipient (605) couplé à la première source d'oxyde nitrique (606), le premier récipient (605) comportant un premier support et un premier réducteur pouvant transformer le premier composé libérant de l'oxyde nitrique en oxyde nitrique et/ou dans lequel la deuxième plate-forme comprend un deuxième récipient couplé à la deuxième source d'oxyde nitrique, le deuxième récipient (605) comportant un deuxième support et un deuxième réducteur pouvant transformer le deuxième composé libérant de l'oxyde nitrique en oxyde nitrique.

12. Système de l'une quelconque des revendications 1 à 11, dans lequel le module de capteurs (612) est couplé à la ligne de distribution et/ou dans lequel le contrôleur (206) est configuré pour recevoir un signal de détection provenant du ou d'au moins un des capteurs d'oxyde nitrique.

13. Système de la revendication 12, dans lequel le contrôleur (206) est configuré pour recevoir un premier signal de détection provenant du ou d'au moins un des capteurs d'oxyde nitrique dans la première ligne de capteurs et un deuxième signal de détection provenant du ou d'au moins un des capteurs d'oxyde nitrique dans la deuxième ligne de capteurs.

14. Système de la revendication 13, dans lequel le contrôleur (206) est configuré pour recevoir un signal d'oxyde nitrique représentant une quantité prédéterminée d'oxyde nitrique devant être distribuée par la ligne de distribution, de préférence dans lequel le contrôleur (206) est configuré pour comparer chacun du premier signal de détection et du deuxième signal de détection au signal d'oxyde nitrique, et pour activer une alerte si le premier signal de détection ou le deuxième final de détection diffère du signal d'oxyde nitrique de plus de 5 % du signal d'oxyde nitrique ou si le premier signal de détection ou le deuxième signal de détection diffère du signal d'oxyde nitrique de plus de 10 % du signal d'oxyde nitrique.

15. Système de l'une quelconque des revendications 1 à 14, dans lequel la première plate-forme (601) comprend une première vanne d'écoulement avant la ligne de distribution, la première vanne d'écoulement ayant une première position configurée pour permettre au premier gaz de passer de la première plate-forme à l'intérieur de la ligne de distribution, et une deuxième position configurée pour permettre au premier gaz de quitter la première plate-forme par le biais d'une première ligne de décharge, ou le système comprenant une première vanne d'écoulement dans la première plate-forme avant la ligne de distribution, la première vanne d'écoulement ayant une position ouverte configurée pour permettre au premier gaz de passer de la première plate-forme à l'intérieur de la ligne de distribution et une position fermée configurée pour empêcher le premier gaz de passer de la première plate-forme à l'intérieur de la ligne de distribution.

16. Système de l'une quelconque des revendications 1 à 15, le système comprenant une première ligne de décharge (615) couplée à la première plate-forme par une première vanne de décharge, la première vanne de décharge ayant une position ouverte configurée pour permettre au premier gaz de passer de la première plate-forme à l'intérieur de la première ligne de décharge (615) et une position fermée configurée pour empêcher le premier gaz de passer à l'intérieur de la première ligne de décharge (615).

17. Système de l'une quelconque des revendications 1 à 16, dans lequel la deuxième plate-forme (602) comprend une deuxième vanne d'écoulement avant la ligne de distribution qui a première position configurée pour permettre au deuxième gaz de passer de la deuxième plate-forme à l'intérieur de la ligne de distribution, et une deuxième position configurée pour permettre au deuxième gaz de quitter la deuxième plate-forme (602) par le biais d'une ligne de décharge (615).

18. Système de l'une quelconque des revendications 1 à 17, comprenant en outre un ensemble d'étalons d'oxyde nitrique comportant au moins un fluides d'étalonnage d'oxyde nitrique, chaque fluide d'étalonnage d'oxyde nitrique ayant une quantité connue différente d'oxyde nitrique, de préférence dans lequel le contrôleur est configuré pour basculer la première vanne d'écoulement de la position ouverte à la position fermée si le ou au moins un des capteurs d'oxyde nitrique dans la première plate-forme (601) détecte une quantité d'oxyde nitrique gazeux dans un des au moins deux fluides d'étalonnage d'oxyde nitrique qui diffère de 10 % ou plus de la quantité connue d'oxyde nitrique dans l'au moins un fluide d'étalonnage d'oxyde nitrique, et/ou dans lequel le contrôleur est configuré pour basculer la deuxième vanne de la position ouverte à la position fermée si le ou au moins un des capteurs de dioxyde d'azote dans la deuxième plate-forme (602) détecte une quantité d'oxyde nitrique gazeux dans un des au moins deux fluides d'étalonnage d'oxyde nitrique qui diffère de 10 % ou plus de la quantité connue d'oxyde nitrique dans l'au moins un fluide d'étalonnage d'oxyde nitrique, et comprenant en outre un ensemble d'étalons de dioxyde d'azote comportant au moins un fluide d'étalonnage de dioxyde d'azote, chaque fluide d'étalonnage de dioxyde d'azote ayant une quantité connue différente de dioxyde d'azote, et/ou un ensemble d'étalons d'oxygène comportant au moins un fluide d'étalonnage d'oxygène, chaque fluide d'étalonnage d'oxygène ayant une quantité connue différente d'oxygène.
